(19) Europäisches Patentamt
European Patent Office
Office européen des brevets

(11) EP 2 279 179 B1

# (12) EUROPEAN PATENT SPECIFICATION

(45) Date of publication and mention of the grant of the patent:
**17.09.2014 Bulletin 2014/38**

(21) Application number: **09755056.0**

(22) Date of filing: **29.05.2009**

(51) Int Cl.:
*C07D 241/04* (2006.01) *C07D 241/02* (2006.01)
*C07D 403/08* (2006.01) *A61K 31/498* (2006.01)

(86) International application number:
**PCT/KR2009/002881**

(87) International publication number:
**WO 2009/145591 (03.12.2009 Gazette 2009/49)**

## (54) PHENYL PIPERAZINE COMPOUNDS, PHARMACEUTICAL COMPOSITION INCLUDING THE SAME, AND USE THEREOF

PHENYLPIPERAZINVERBINDUNGEN, PHARMAZEUTISCHE ZUSAMMENSETZUNG DAMIT UND VERWENDUNG DAVON

COMPOSÉS DE PHÉNYL-PIPÉRAZINE, COMPOSITION PHARMACEUTIQUE LES COMPRENANT, ET UTILISATION ASSOCIÉE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **29.05.2008 US 128999**

(43) Date of publication of application:
**02.02.2011 Bulletin 2011/05**

(73) Proprietor: **SK Biopharmaceuticals Co., Ltd.**
**Jongro-gu**
**Seoul**
**110-110 (KR)**

(72) Inventors:
- **KIM, Yonggil**
  **Daejon 305-761 (KR)**
- **CHO, Nahmryune**
  **Daejeon 305-808 (KR)**
- **KIM, Yunhee**
  **Anyang-si**
  **Gyeonggi-do 431-050 (KR)**
- **HEO, Joon**
  **Daejeon 305-762 (KR)**
- **DONG, Seonmin**
  **Goyang-si**
  **Gyeonggi-do 410-908 (KR)**
- **JI, Mi Kyung**
  **Daejeon 305-712 (KR)**
- **CHA, Man-Young**
  **Daejeon 300-717 (KR)**
- **LEE, Kiho**
  **Daejeon 305-509 (KR)**

(74) Representative: **Kling, Simone**
**Lavoix Munich**
**Bayerstrasse 83**
**80335 München (DE)**

(56) References cited:
**WO-A1-98/18797** **WO-A1-98/42692**
**WO-A2-2004/112729**

- **SUKALOVIC, V. ET AL.: 'Synthesis, dopamine D2 receptor binding studies and docking analysis of 5-[3-(4-arylpiperazin-1-yl)propyl]-1H-benzimidazole, 5-[2-(4-arylpiperazin-1-yl)ethoxy]-1H-benzimidazole and their analogs' EUROPEAN JOURNAL OF MEDICINAL CHEMISTRY vol. 40, 2005, pages 481 - 493, XP004888399**
- **KOWALSKI, P. ET AL.: 'The Synthesis of Cyclic and Acyclic Long-chain Arylpiperazine Derivatives of Salicylamides as Serotonin Receptor Ligands' JOURNAL OF HETEROCYCLIC CHEMISTRY. vol. 39, no. 1, January 2008, pages 209 - 214, XP008144197**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to novel piperazine derivatives or pharmaceutically acceptable salts thereof, a process for preparing the same, and in particular, a high binding for Serotonin 1A(5-hydroxytryptamine; 5-HT1A) receptor, a pharmaceutical composition for treatment and/or prevention of depression and anxiety including an effective amount of the piperazine compound, and said composition for use in a method of treating depression, anxiety and other conditions related to 5-HT1A receptor in a mammal.

### BACKGROUND OF THE INVENTION

Depression affects approximately 14 million people in the United States and 340 million people worldwide, making it one of the leading causes of disability [Kessler RC. Et al., JAMA, 2003, 289, 3095-3105]. Depression is also associated with high rates of relapse, recurrence, disability, and death [Hirschfield RM., et al., JAMA, 1997, 277, 333-340; Keller MB., J. Psychopharmacol.1996, 10(suppl 1);41-44].

The role of serotonin in the treatment of depressive and anxiety disorders is underscored by the therapeutic action of selective 5-HT reuptake inhibitors acting to enhance the degree of activation of various 5-HT receptor subtypes [Blier P. and Ward M., Biol. Psychiatry, 2003, 53,193-203]. Selective serotonin reuptake inhibitors(SSRIs) have had significant success in treating depression and related disorders and have become among the most prescribed drugs since 1980s. But, the use of SSRIs leads to the indiscriminate activation of all serotonin receptors and it can be understood as undesirable actions of serotonin in undesirable pathways at undesirable receptor subtypes [Stahl SM., 2008, Essential Psychopharmacology(3rd ed.), 531]. Although there are various treatment options for depressive and anxiety disorders, a need still exists for new drugs with improved tolerability and adequate efficacy.

In contrast to the SSRIs, the 5-HT1A agonist or partial agonist acts directly on postsynaptic serotonin receptors to increase serotonergic neurotransmission. And, the 5-HT1A partial agonists, Buspirone and Gepirone[Robinson DS., et al., J. Clin. Psychopharmacol., 1990, 10(3), 67S-76S; Bielski RJ., J. Clin. Psychiatry, 2008, 69(4), 571-577], and 5-HT1A agonist, Flexinoxan [Grof P., International Clin. Psychopharmacology, 1993, 8(3), 167-172], have shown efficacy in clinical trials for the treatment of depression. Moreover, because of their unique pharmacological profile, 5-HT1A agonists possess theoretic advantages in the treatment of major depression relative to other classes of antidepressants. In particular, these drugs are not expected to produce weight gain, sedation, or sexual dysfunctions, which are often encountered with some of the antidepressants introduced in the last 10 years [Blier P. and Ward M., Biol. Psychiatry, 2003, 53, 193-203].

Many reports have disclosed that phenyl piperazine compounds are effectively used for controlling depression and anxiety.

For example, US 5,578,596 discloses that the 2-alkoxy-5,6,7,8-tetrahydroquinoxaline derivatives have a strong affinity for serotonin 1A receptor and are useful for preventing and treating serotonergic neuron-related disease.

WO 98/42692 discloses phenyl piperazine derivatives for use in the treatment of diseases associated to the modulation of the 5-HT1A receptor.

These compounds are found to be very effective as therapeutical medicines for managing depression and anxiety.

Active research and development efforts have continued to be directed to the application of phenyl piperazine compounds for the treatment of depression and anxiety.

### SUMMARY OF THE INVENTION

In one aspect, the invention provides a piperazine compound represented by Formula (I) or pharmaceutically acceptable salts thereof:

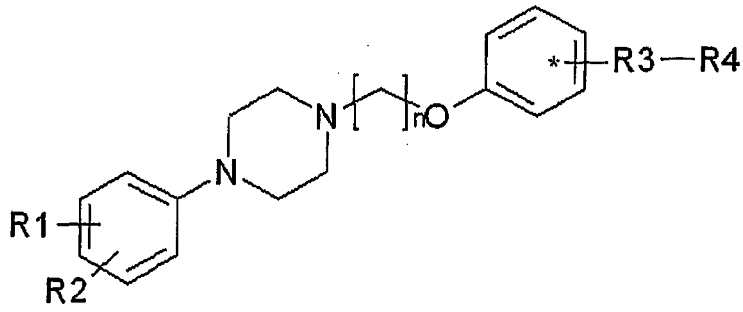

(I)

wherein

n is an integer from 2 to 6;
R1 and R2 are the same or different and are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms;
R3 is a C1-C2 alkylene; and
R4 is selected from the group consisting of:

(a) a C2-C6 dialkylamine,
(b) a 5 to 9-membered aromatic amine wherein the ring is independently substituted with at least of hydrogen, a C1-C6 aliphatic alkyl, a C3 to C10 cyclic alkyl, a C6-C10 aryl group and a halogen,
(c) a 5 to 9-membered heteroaromatic amine comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen,
(d) a 5 to 9-membered heterocyclic ring comprising at least a nitrogen atom wherein the ring is independently substituted with at least one of hydrogen, a C1-C6 aliphatic alkyl, a C3 to C10 cyclic alkyl, a C6-C10 aryl group and a halogen, and
(e) a 5 to 9-membered heteroaromatic ring comprising at least a nitrogen atom wherein the ring is independently substituted with at least one of hydrogen, a C1-C6 aliphatic alkyl, a C3 to C10 cyclic alkyl, a C6-C10 aryl group and a halogen; and

R4 is connected to R3 group through a nitrogen atom therein.

In another aspect, the invention provides a pharmaceutical composition comprising:

(A) an effective amount of a piperazine compound represented by Formula (I):

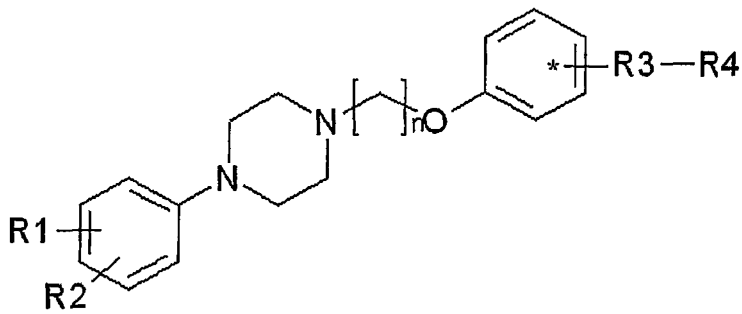

(I)

or pharmaceutically acceptable salts thereof, wherein

n is an integer from 2 to 6;
R1 and R2 are the same or different and are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms;
R3 is a C1-C2 alkylene; and
R4 is selected from the group consisting of:

(a) a C2-C6 dialkylamine,

(b) a 5 to 9-membered aromatic amine wherein the ring is independently substituted with at least one of hydrogen, a C1-C6 aliphatic alkyl, a C3 to C10 cyclic alkyl, a C6-C10 aryl group and a halogen,

(c) a 5 to 9-membered heteroaromatic amine comprising at least a nitrogen atom as a ring constituent wherein the ring is independently substituted with at least one of hydrogen, a C 1-C6 aliphatic alkyl, a C3 to C10 cyclic alkyl, a C6-C10 aryl group and a halogen,

(d) a 5 to 9-membered heterocyclic ring comprising at least a nitrogen atom wherein the ring is independently substituted with at least of hydrogen, a C1-C6 aliphatic alkyl, a C3 to C10 cyclic alkyl, a C6-C10 aryl group and a halogen, and

(e) a 5 to 9-membered heteroaromatic ring comprising at least a nitrogen atom wherein the ring is independently substituted with at least one of hydrogen, a C1-C6 aliphatic alkyl, a C3 to C10 cyclic alkyl, a C6-C10 aryl group and a halogen; and

R4 is connected to R3 group through a nitrogen atom therein; and

(B) a pharmaceutically acceptable carrier or excipient.

In yet another aspect, the invention provides a piperazine compound of formula (I) for use in a method for treating at least one of depression and anxiety in a mammal comprising administering to a mammal in need thereof an effective amount of said compound:

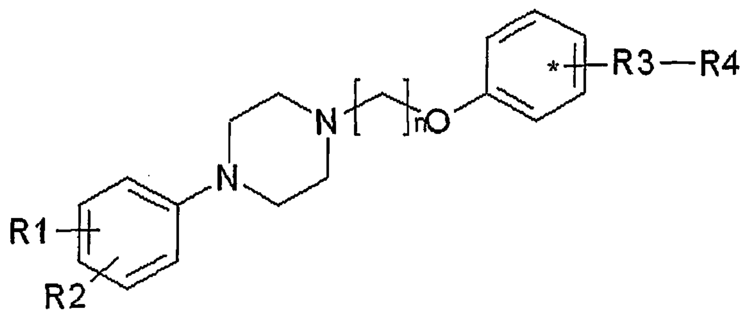

(I)

or pharmaceutically acceptable salts thereof, wherein

n is an integer from 2 to 6;

R1 and R2 are the same or different and are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms;

R3 is a C1-C2 alkylene; and

R4 is selected from the group consisting of:

(a) a C2-C6 dialkylamine,

(b) a 5 to 9-membered aromatic amine wherein the ring is independently substituted with at least one of hydrogen, a C1-C6 aliphatic alkyl, a C3 to C10 cyclic alkyl, a C6-C10 aryl group and a halogen,

(c) a 5 to 9-membered heteroaromatic amine comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen,

(d) a 5 to 9-membered heterocyclic ring comprising at least a nitrogen atom wherein the ring is independently substituted with at least one of hydrogen, a C1-C6 aliphatic alkyl, a C3 to C10 cyclic alkyl, a C6-C10 aryl group and a halogen, and

(e) a 5 to 9-membered heteroaromatic ring comprising at least a nitrogen atom wherein the ring is independently substituted with at least one of hydrogen, a C1-C6 aliphatic alkyl, a C3 to C10 cyclic alkyl, a C6-C10 aryl group and a halogen; and

R4 is connected to R3 group through a nitrogen atom therein.

DETAILED DESCRIPTION OF THE INVENTION

Before any embodiments of the invention are explained in detail, it is to be understood that the invention is not limited in its application to the details of construction and the arrangement of components set forth in the following description. The invention is capable of other embodiments and of being practiced or of being carried out in various

ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It also is understood that any numerical range recited herein includes all values from the lower value to the upper value. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this application.

Throughout the specification and the appended claims, a given chemical formula or name shall encompass all stereo and optical isomers and racemates thereof as well as mixtures in different proportions of the separate enantiomers, where such isomers and enantiomers exist, as well as pharmaceutically acceptable salts thereof and solvates thereof such as, for instance, hydrates. Isomers may be separated using conventional techniques, e.g. chromatography or fractional crystallization. The enantiomers may be isolated by separation of racemates for example by fractional crystallization, resolution, or HPLC. The diastereomers may be isolated by separation of isomer mixtures for instance by fractional crystallization, HPLC, or flash chromatography. A "heteroaromatic ring" or a "heterocylic ring" means a ring containing at least one heteroatom. A "heteroatom" means an atom other than carbon in the ring of a heterocylic group or a heteroaromatic group. Heteroatoms may be at least one of oxygen, sulfur, and nitrogen atoms. Groups containing more than one heteroatom may contain different heteroatoms.

The piperazine compounds of the invention have Chemical Formula (I) or pharmaceutically acceptable salts thereof.

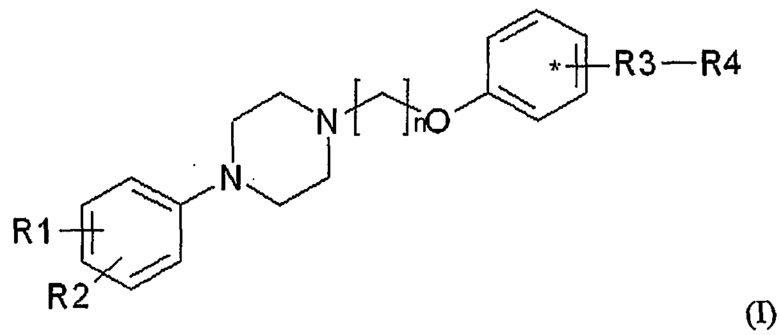

(I),

wherein

n is an integer from 2 to 6;
R1 and R2 are the same or different and are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms;
R3 is a C1-C2 alkylene; and
R4 is selected from the group consisting of:

(a) a C2-C6 dialkylamine,
(b) a 5 to 9-membered aromatic amine wherein the ring is independently substituted with at least of hydrogen, a C1-C6 aliphatic alkyl, a C3 to C10 cyclic alkyl, a C6-C10 aryl group and a halogen,
(c) a 5 to 9-membered heteroaromatic amine comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen,
(d) a 5 to 9-membered heterocyclic ring comprising at least a nitrogen atom wherein the ring is independently substituted with at least one of hydrogen, a C1-C6 aliphatic alkyl, a C3 to C10 cyclic alkyl, a C6-C10 aryl group and a halogen, and
(e) a 5 to 9-membered heteroaromatic ring comprising at least a nitrogen atom wherein the ring is independently substituted with at least one of hydrogen, a C1-C6 aliphatic alkyl, a C3 to C10 cyclic alkyl, a C6-C10 aryl group and a halogen; and

R4 is connected to R3 group through a nitrogen atom therein.

In one embodiment, R1 and R2 are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms. For example, the halogen may be F, Cl, Br, or I. The alky group may be methyl, ethyl, n-propyl, isopropyl, n-butyl, iso-butyl, sec-butyl, and t-butyl, and particularly may be methyl, ethyl,

propyl, isopropyl, and tertiary butyl. Unless otherwise stated or indicated, the term "alkoxy" denotes a group O-alkyl, wherein alkyl is methyl, ethyl, n-propyl, isopropyl, n-butyl, iso-butyl, sec-butyl, or t-butyl.

Unless otherwise stated or indicated, the term "halogen" shall mean fluorine, chlorine, bromine, or iodine.

Examples of R4 include, without limitation, dimethyl amine, diethylamine, dipropylamine, methylethyl amine, methylpropyl amine, methylbutyl amine, and ethylpropy amine.

When R4 is a 5 to 9-membered aromatic amine or a 5 to 9-membered heteroaromatic amine, R4 is connected to R3 through a nitrogen atom in an amino group.

R4 may be a 5 to 9-membered aromatic amine wherein the ring is independently substituted with at least one of hydrogen, a C1-C6 aliphatic alkyl, a C3 to C10 cyclic alkyl, a C6-C10 aryl group and a halogen. In one embodiment, examples of a 5 to 9-membered aromatic amine in R4 is a 5 or 6-membered aromatic amine substituted with at least one of hydrogen, a C1-C6 alkyl, and a halogen, and particularly, a 6-membered aromatic amine substituted with hydrogen, such as aniline.

R4 may be a 5 to 9-membered heteroaromatic amine containing at least a nitrogen atom where the ring is independently substituted with at least one of hydrogen, a C1-C6 aliphatic alkyl, a C3 to C10 cyclic alkyl, a C6-C10 aryl group, and a halogen. In one embodiment, examples of a 5 to 9-membered heteroaromatic amine include a 5 or 6-membered heteroaromatic amine substituted with at least one of a C1-C6 alkyl and a halogen, wherein the heteroaromatic amine comprises at least two heteroatoms as ring constituents where a first heteroatom is N and a second heteroatom is independently selected from the group consisting of N, O, and S.

The 5-membered heteroaromatic amine may have a structure (II) selected from the group consisting of:

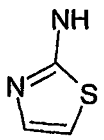 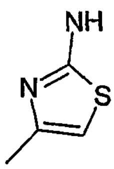 and 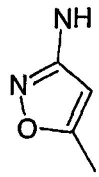 (II)

When R4 is a 5 to 9-membered heterocyclic ring or a 5 to 9-membered heteroaromatic ring, R4 may be connected to R3 through a nitrogen atom contained in the ring as a heteroatom of the 5 to 9-membered heterocylic ring or heteroaromatic ring.

R4 may be a 5 to 9-membered heterocyclic ring containing at least a nitrogen atom where the ring is independently substituted with at least one of hydrogen, a C1-C6 aliphatic alkyl, a C3 to C10 cyclic alkyl, a C6-C10 aryl group, and a halogen. Examples of a 5 to 9-membered heterocylic ring include, without limitation, a 5 or 6-membered heterocylic ring having a structure (III) selected from the group consisting of:

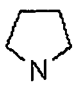 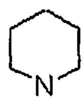 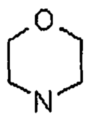 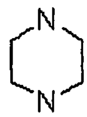 and 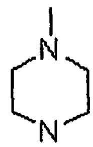 (III)

R4 may be a 5 to 9-membered heteroaromatic ring containing at least a nitrogen atom where the ring is independently substituted with at least one of hydrogen, a C1-C6 aliphatic alkyl, a C3 to C10 cyclic alkyl, a C6-C10 aryl group, and a halogen. Examples of a heteroaromatic ring include, without limitation, an azole compound such as diazole, triazole, tetraazol, benzotriazole, imidazole, pyrazole, benzimidazloe or indazole. The azole ring may be independently substituted with at least one of hydrogen, a C1-C6 aliphatic alkyl, a C3 to C10 cyclic alkyl, a C6-C10 aryl group, and a halogen. The azole compound may have a structure (IV) selected from the group consisting of:

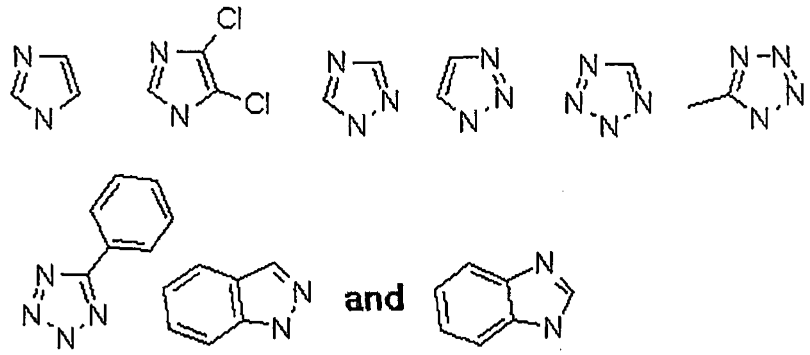

and

(IV)

Examples of compounds having Chemical Formula (I) include, without limitation, 1-[4-(4-imidazol-1-ylmethyl-phenoxy)-butyl]-4-(2-methoxy-phenyl)-piperazine, 1-(2-methoxy-phenyl)-4-[4-(4-[1,2,4]triazol-1-ylmethyl-phenoxy)-butyl]-piperazine, 1-(2-methoxy-phenyl)-4-[4-(4-tetrazol-1-ylmethyl-phenoxy)-butyl]-piperazine, 1-(2-methoxy-phenyl)-4-[4-(4-tetrazol-2-ylmethyl-phenoxy)-butyl]-piperazine, 1-[4-(3-imidazol-1-ylmethyl-phenoxy)-butyl]-4-(2-methoxy-phenyl)-piperazine- hydrochloride, 1-(2-methoxy-phenyl)-4-[4-(3-[1,2,4]triazol-1-ylmethyl-phenoxy)-butyl]-piperazine hydrochloride, 1-(2-methoxy-phenyl)-4-[4-(3-tetrazol-1-ylmethyl-phenoxy)-butyl]-piperazine hydrochloride, 1-(2-methoxy-phenyl)-4-[4-(3-tetrazol-2-ylmethyl-phenoxy)-butyl]-piperazine hydrochloride, 1-(4-{4-[4-(2-methoxy-phenyl)-piperazin-1-yl]-butoxy}-benzyl)-1H-benzoimidazole, 1-(2-Methoxy-phenyl)-4-{4-[4-(2-methyl-imidazol-1-ylmethyl)-phenoxy]-butyl}-piperazine, 1-(2-methoxy-phenyl)-4-[4-(4-[1,2,3]triazol-2-ylmethyl-phenoxy)-butyl]-piperazine, 1-(2-methoxy-phenyl)-4-[4-(4-[1,2,3]triazol-1-ylmethyl-phenoxy)-butyl]-piperazine, 1-(4-{4-[4-(2-Methoxy-phenyl)-piperazin-1-yl]-butoxy}-benzyl)-1H-indazole, 1-(4-{4-[4-(2-Methoxy-phenyl)-piperazin-1-yl]-butoxy}-benzyl)-1H-benzotriazole, 1-(2-methoxy-phenyl)-4-{4-[4-(5-phenyl-tetrazol-2-ylmethyl)-phenoxy]-butyl}-piperazine, 1-(2-methoxy-phenyl)-4-{4-[4-(5-phenyl-tetrazol-1-ylmethyl)-phenoxy]-butyl}-piperazine, 1-(2-methoxy-phenyl)-4-{4-[4-(5-methyl-tetrazol-2-ylmethyl)-phenoxy]-butyl}-piperazine, 1-(2-methoxy-phenyl)-4-{4-[4-(5-methyl-tetrazol-1-ylmethyl)-phenoxy]-butyl}-piperazine,(4-{4-[4-(2-methoxy-phenyl)-piperazin-1-yl]-butoxy}-benzyl)-dimethyl-amine di-hydrochloride, 1-(2-methoxy-phenyl)-4-[4-(4-piperidin-1-ylmethyl-phenoxy)-butyl]-piperazine, (4-{4-[4-(2-methoxy-phenyl)-piperazin-1-yl]-butoxy}-benzyl)-phenyl-amine, (4-{4-[4-(2-methoxy-phenyl)-piperazin-1-yl]-butoxy}-benzyl)-thiazol-2-yl-amine, 1-(2-chloro-phenyl)-4-[4-(4-[1,2,4]triazol-1-ylmethyl-phenoxy)-butyl]-piperazine, 1-(2-methoxy-phenyl)-4-[4-(3-[1,2,3]triazol-2-ylmethyl-phenoxy)-butyl]-piperazine, 1-(2-Methoxy-phenyl)-4-[4-(3-[1,2,3]triazol-1-ylmethyl-phenoxy)-butyl]-piperazine, 1-(2-Methoxy-phenyl)-4-[4-(3-[1,2,3]triazol-2-ylmethyl-phenoxy)-butyl]-piperazine, 1-(2-methoxy-phenyl)-4-{4-[3-(5-methyl-tetrazol-1-ylmethyl)-phenoxy]-butyl}-piperazine, (3-{4-[4-(2-methoxy-phenyl)-piperazin-1-yl]-butoxy}-benzyl)-dimethyl-amine, 4-(3-{4-[4-(2-methoxy-phenyl)-piperazin-1-yl]-butoxy}-benzyl)-morpholine, 1-{4-[4-(4,5-dichloro-imidazol-1-ylmethyl)-phenoxy]-butyl}-4-(2-methoxy-phenyl)-piperazine, 1-(2-chloro-phenyl)-4-[4-(4-tetrazol-2-ylmethyl-phenoxy)-butyl]-piperazine, 4-(4-{4-[4-(2-methoxy-phenyl)-piperazin-1-yl]-butoxy}-benzyl)-morpholine, (4-{4-[4-(2-Chloro-phenyl)-piperazin-1-yl]-butoxy}-benzyl)-dimethyl-amine, 1-(3-fluoro-phenyl)-4-[4-(4-[1,2,4]triazol-1-ylmethyl-phenoxy)-butyl)-piperazine, 1-(2-methoxy-phenyl)-4-[4-(4-4-methylpiperazine-1-ylmethyl-phenoxy)-butyl]-piperazine, , 1-(2,3-dichloro-phenyl)-4-[4-(4-tetrazol-2-ylmethyl-phenoxy)-butyl]-piperazine, 1-(2,3-Dichloro-phenyl)-4-{4-[4-(5-methyl-tetrazol-1-ylmethyl)-phenoxy]-butyl}-piperazine, 1-(2,3-Dichloro-phenyl)-4-{4-[4-(5-methyl-tetrazol-2-ylmethyl)-phenoxy]-butyl}-piperazine, (4-{4-[4-(2,3-dichloro-phenyl)-piperazin-1-yl]-butoxy}-benzyl)-dimethyl-amine, 4-(4-{4-[4-(2,3-Dichloro-phenyl)-piperazin-1-yl]-butoxy}-benzyl)-morpholine, 1-(2-methoxy-phenyl)-4-[4-(4-4-methylpiperazine-1-ylmethyl-phenoxy)-butyl]-piperazine, 1-(2-chloro-phenyl)-4-[4-(4-[1,2,3]triazol-1-ylmethyl-phenoxy)-butyl]-piperazine, (4-{4-[4-(2-Methoxy-phenyl)-piperazin-1-yl]-butoxy}-benzyl)-[1,3,4]thiadiazol-2-yl-amine, 1-(2,3-dichloro-phenyl)-4-[4-(4-[1,2,3]triazol-1-ylmethyl-phenoxy)-butyl]-piperazine, (4-{4-[4-(2-Methoxy-phenyl)-piperazin-1-yl]-butoxy}-benzyl)-(5-methyl-isoxazol-3-yl)-amine, (4-{4-[4-(2-methoxy-phenyl)-piperazin-1-yl]-butoxy}-benzyl)-(5-methyl-isoxazol-3-yl)-amine, 1-{4-[4-(2-imidazol-1-yl-ethyl)-phenoxy]-butyl}-4-(2-methoxy-phenyl)-piperazine, 1-(2-methoxy-phenyl)-4-{4-[4-(2-[1,2,4]triazol-1-yl-ethyl)-phenoxy]-butyl}-piperazine, 1-(2-methoxy-phenyl)-4-{4-[4-(2-[1,2,3]triazol-2-yl-ethyl)-phenoxy]-butyl}-piperazine, 1-(2-methoxy-phenyl)-4-{4-[4-(2-[1,2,3]triazol-l-yl-ethyl)-phenoxy]-butyl}-piperazine, 1-(2-methoxy-phenyl)-4-{4-[4-(2-tetrazol-2-yl-ethyl)-phenoxy]-butyl}-piperazine, 1-(2-methoxy-phenyl)-4-{4-[4-(2-tetrazol-1-yl-ethyl)-phenoxy]-butyl}-piperazine, 1-(2-Methoxy-phenyl)-4-(4-{4-[2-(5-methyl-tetrazol-1-yl)-ethyl]-phenoxy}-butyl)-piperazine, 1-(2-Methoxy-phenyl)-4-(4-{4-[2-(5-methyl-tetrazol-2-yl)-ethyl]-phenoxy}-butyl)-piperazine, 2-{4-[4-(4-[1,2,3]-triazol-1-ylmethyl-phenoxy)-butyl]-piperazin-1-yl}-benzonitrile, and 1-(2-chloro-phenyl)-4-[4-(4-[1,2,3]triazol-2-ylmethyl-phenoxy)-butyl]-piperazine.

A "pharmaceutically acceptable salt", where such salts are possible, includes both pharmaceutically acceptable acid and base addition salts. A suitable pharmaceutically acceptable salt of a compound of Formula I is, for example, an acid-addition salt of a compound of Formula I that is sufficiently basic, for example an acid-addition salt with an

inorganic or organic acid such as hydrochloric, hydrobromic, sulphuric, trifluoroacetic, citric or maleic acid; or, for example, a salt of a compound of Formula I that is sufficiently acidic, for example an alkali or alkaline earth metal salt such as a sodium, calcium, or magnesium salt, or an ammonium salt, or a salt with an organic base such as methylamine, dimethylamine, trimethylamine, piperidine, morpholine, or tris-(2-hydroxyethyl)amine.

A method for preparing the above piperazine derivative, represented by chemical Formula (I) will be described below in detail.

First, phenols of Formula (VI) as starting material are reacted with dihalide alkanes represented by Formula (V) to synthesize halogenized compounds represented by Formula (VII):

$X\text{-}(CH_2)n\text{-}X$ (V)

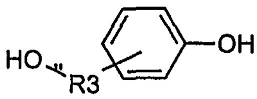

(VI)

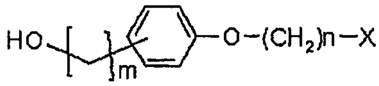

(VII)

wherein n is the same as defined above and X is a halogen such as, without limitation, chloride and bromide.

The halogenized compound represented by Formula (VII) is reacted with phenyl piperazines of Formula (VIII) to obtain the compounds having hydroxy group represented by the Formula (IX):

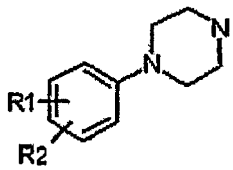

(VIII)

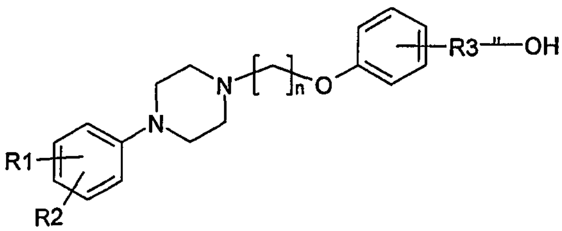

(IX)

wherein R1, R2, n and R3 are the same as defined above.

This procedure is summarized as set forth in Reaction Scheme (I) below.

## Reaction Scheme I

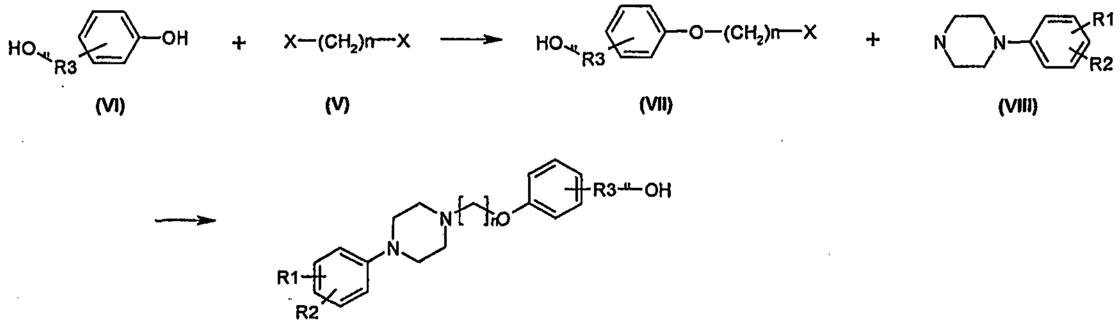

(VI) (V) (VII) (VIII)

(IX)

Details of the reaction conditions described in Reaction Scheme I may be as follows. For the conversion of the phenols (VI) to the halogenized compound (VII), the concentration of the phenols (VI) may be about 0.005 to about 0.1 moles, with a dihalide alkane (V) ranging from about 2.0 to about 3.0 equivalents and an organic or inorganic base from about 3.0 to about 4.0 equivalents. This reaction may be refluxed. The resulting product may be purified by column chromatography. For the conversion of the halogenized compounds (VII) to the compound having hydroxy group (IX), a mixture of (VII) and substituted phenylpiperazine (VIII) may be refluxed in about 30ml of acetonitrile for about 6h. For this coupling reaction, an ethereal solvent such as diethyl ether and tetrahydrofuran, a halogenated hydrocarbon solvent such as dichloromethane and chloroform, or a mixture thereof may be used.

Then, the hydroxy compounds having hydroxy group represented by Formula (IX) are reacted with thionyl chloride and then with dialkylamines, aromatic amine, heteroaromatic amine, heterocyclic ring, or heteroaromatic ring groups to produce the phenyl piperazine compounds represented by Formula (I):

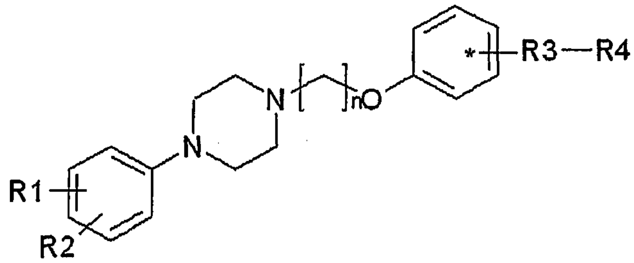

(I)

wherein,

R1, R2, R3, R4, and n are as previously defined.

The pharmaceutically acceptable salts thereof represented by Formula (XI) as below can be obtained by treating a phenyl piperazine compound of Formula (I) with an anhydrous acid in a solution without further purification.

This procedure is summarized as set forth in Reaction Scheme (II) below.

## Reaction Scheme II

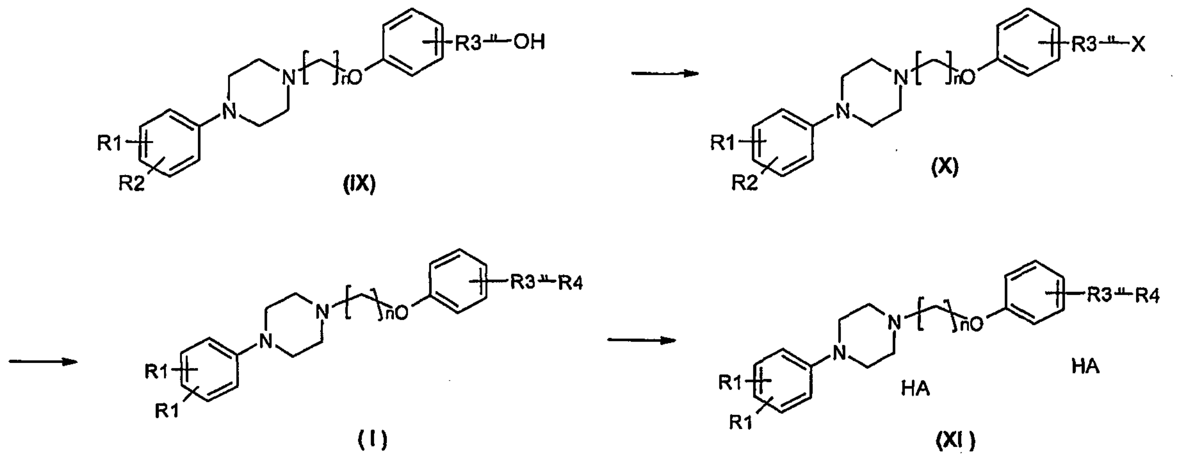

(IX) (X)

(I) (XI)

Details of the reaction conditions described in Reaction Scheme II are as follows. For the conversion of the compounds having hydroxy group (IX) to the compound (X), the concentration of the compounds having hydroxy group (IX) may be about 0.005 to about 0.1 moles with thionyl chloride ranging from about 1.0 to about 1.5 equivalents. This reaction may be carried out at a temperature of about 0°C. The resulting product may be purified by column chromatography. The resulting compound (X) may be treated with about 2.0 to about 3.0 equivalents of dialkylamines, aromatic amine, heteroaromatic amine, heterocyclic ring, or heteroaromatic ring at a reflux to give the compound of the General Formula (I). For this coupling reaction, tetrahydrofuran, acetonitrile, a halogenated hydrocarbon solvent such as dichloromethane and chloroform, or a mixture thereof may be used.

In Reaction Scheme II, HA represents an acid that is capable of forming a pharmacologically useful salt with the basic nitrogen atom. Specific examples of the anhydrous acid used for the preparation of the compound (XI) include, without limitation, hydrochloric acid, sulfuric acid, phosphoric acid, acetic acid, benzoic acid, citric acid, malonic acid, salicylic acid, malic acid, fumaric acid, oxalic acid, succinic acid, tartaric acid, lactic acid, gluconic acid, ascorbic acid, maleic acid, aspartic acid, benzene sulfonic acid, methane sulfonic acid, ethane sulfonic acid, hydroxymethane sulfonic acid, hydroxyethane sulfonic acid, and the like. For additional acids, one can refer to "Pharmaceutical Salts", J. Pharm. Sci., 1977; 66(1): 1-19. This preparation may be executed in a reaction media that can be exemplified by an ethereal solvent such as tetrahydrofuran, an alcoholic solvent such as methanol, an ester solvent such as ethyl acetate, a halogenated hydrocarbon solvent, and mixtures thereof. In one embodiment, an ethereal solvent is recommended as an addition solution, including ethyl ether, propyl ether, isopropyl ether, butyl ether, and isobutyl ether. The concentration of the compound (I) may be in the order of about 0.01 to about 5 moles.

In another embodiment of the present invention, the present invention provides a pharmaceutical composition including an effective amount of phenyl piperazine compounds represented by Formula (I) for treating at least one of depression and anxiety.

In a further embodiment of the present invention, the present invention provides piperazine compounds represented by formula (I) for use in a method of treating at least one of depression and anxiety in a mammal comprising administering to a mammal in need thereof an effective amount of said compounds, alone or in combination with a pharmaceutically acceptable carrier or excipient.

The activity of the compounds of the present invention was examined through the Mouse Tail Suspension and Marble Burying Test.

In therapeutic use as agents for depression and anxiety the compounds of the present invention are used, alone or in combination with a pharmaceutically acceptable carrier or excipient.

The compounds of the present invention may be administered orally or parentally, neat or in combination with conventional pharmaceutical carriers. Applicable solid carriers can include, without limitation, one or more substances which may also act as flavoring agents, lubricants, solubilizers, suspending agents, fillers, glidants, compression aids, binders, tablet-disintegrating agents, or encapsulating materials. In powders, the carrier may be a finely divided solid that may be in admixture with the finely divided active ingredient. In tablets, the active ingredient may be mixed with a carrier having suitable compression properties in suitable proportions and compacted in the shape and size desired. The powders and tablets may contain up to about 99% of the active ingredient.

Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes, and ion exchange resins. Liquid carriers may be used in preparing solutions, suspensions, emulsions, syrups, and elixirs.

The active ingredient of this invention may be dissolved or suspended in a pharmaceutically acceptable liquid

carrier such as water, an organic solvent, a mixture of both, or pharmaceutically acceptable oils or fats. The liquid carrier may contain other suitable pharmaceutical additives such as solubilizers, emulsifiers, buffers, preservatives, sweeteners, flavoring agents, suspending agents, thickening agents, colors, viscosity regulators, stabilizers, or osmo-regulators. Suitable examples of liquid carriers for oral and parenteral administration include water (particularly containing additives as above, e.g., cellulose derivativessuch as, without limitation, a sodium carboxymethyl cellulose solution), alcohols (including, without limitation, monohydric alcohols and polyhydric alcohols, e.g., glycols) and their derivatives, and oils (e.g., without limitation, fractionated coconut oil and arachis oil).

For parenteral administration the carrier can also be an oily ester such as ethyl oleate and isopropyl myristate. Sterile liquid carriers are used in sterile liquid form compositions for parenteral administration. Liquid pharmaceutical compositions that are sterile solutions or suspensions can be utilized by, for example, intramuscular, intraperitoneal, or subcutaneous injection. Sterile solutions can also be administered intravenously.

Oral administration may be either in liquid or solid composition form. In one embodiment, the pharmaceutical compositions containing the present compounds are in unit dosage form, e.g., as tablets or capsules. In such form, the composition may be sub-divided in unit dosages containing appropriate quantities of the active ingredients. The unit dosage forms can be packaged compositions, for example, packaged powders, vials, ampoules, prefilled syringes or sachets containing liquids. Alternatively, the unit dosage form can be, for example, a capsule or tablet itself, or it can be the appropriate number of any such compositions in package form. The therapeutically effective dosage to be used may be varied or adjusted by the physician and generally ranges from about 0.5 mg to about 750 mg, according to the specific condition(s) being treated and the size, age, and response pattern of the patient.

The compounds of the present invention may be administered to patients at a dosage of from about 0.7 to about 7000 mg per day. For example, for a normal human adult with a body weight of approximately 70kg, the administration amount is translated into a daily dose of about 0.01 to about 100 mg per kg of body weight. The specific dosage employed, however, may vary depending upon the requirements of the patient, the severity of the patient's condition, and the activity of the compound. The determination of optimum dosages for a particular situation may be clinically done and is within the skill of the art.

A better understanding of the present invention may be obtained in light of the following examples to illustrate, but are not to be construed to limit, the present invention.

**Example 1**

**1-[4-(4-imidazol-1-ylmethyl-phenoxy)-butyl]-4-(2-methoxy-phenyl)-piperazine.**

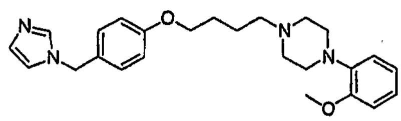

A mixture of 4-hydroxybenzaldehyde (5mmol), 1-bromo-4-chlorobutane (5mmol), and potassium carbonate (15mmol) was refluxed in 100ml of acetone for 6h. This solution was then concentrated on a rotary evaporator and diluted with ethyl acetate. This mixture was then washed with brine, and the resulting organic layer was dried and purified by column chromatography. This was dissolved in methanol (50ml) and was added with sodium borohydride (10mmol) at 25°C. This mixture was stirred at 25°C for 2h. This solution was then concentrated in a rotary evaporator and diluted with methylene chloride, then washed with brine, and the resulting organic layer was dried and concentrated in vacuo. The crude product was dissolved in isopropanol (50ml) and was added with 1-(2-methoxyphenyl)-piperazine (5mmol), sodium carbonate (15mmol), and potassium iodide (5mmol) at 25°C, and the reaction mixture was refluxed for 12h. This solution was then concentrated on a rotary evaporator and diluted with methylene chloride, then washed with brine, and the resulting organic layer was dried and purified by column chromatography. This product was dissolved in CHCl3 (30ml) and was added with pyridine and thionyl chloride (5mmol) at 0°C, then stirred at room temperature for 16h, and water was added to terminate the reaction. The organic layer was extracted 3 times with dichloromethane, dried, and concentrated in vacuo. The residue was purified by column chromatography. This was dissolved in acetonitrile (30ml) and was added with imidazole (6mmol), potassium carbonate (15mmol), and potassium iodide (5mmol) at 25°C, and the reaction mixture was refluxed for 12h. This solution was then concentrated in a rotary evaporator and diluted with methylene chloride, then washed with brine, and the resulting organic layer was dried and purified by column chromatography. The resulting 1-[4-(4-imidazole-1-ylmethyl-phenoxy)-butyl]-4-(2-methoxy-phenyl)-piperazine was dissolved in dichloromethane and the solution was treated with a solution of HCl in ethyl ether. The resulting precipitate was filtered to give 1-[4-(4-imidazole-1-ylmethyl-phenoxy)-butyl]-4-(2-methoxy-phenyl)-piperazine hydrochloride.

$^1$H-NMR ($CDCl_3$, 200MHz) δ 11.1 (br, 1H), 7.5 (s, 1H), 7.3 (s, 1H), 7.2(m, 2H), 6.9(m, 7H), 5.0(s, 2H), 4.0(t, 2H), 3.8 (s, 3H), 3.2(m, 4H), 2.7(m, 4H), 2.5(t, 2H), 1.8(m, 4H)

**Example 2**

**1-(2-methoxy-phenyl)-4-[4-(4-[1,2,4]triazol-1-ylmethyl-phenoxy)-butyl]-piperazine.**

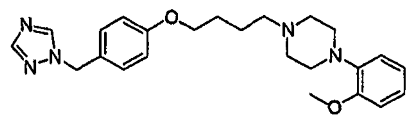

The procedure given in Example 1 was followed using 1H-1,2,4-triazole as reactants, instead of imidazole, to give 1-(2-methoxy-phenyl)-4-[4-(4-[1,2,4]triazole-1-ylmethyl-phenoxy)-butyl]-piperazine.

$^1$H-NMR ($CDCl_3$, 200MHz) δ 8.1 (s, 1H), 8.0 (s, 1H), 7.2(m, 2H), 6.9(m, 6H), 5.2(s, 2H), 4.0(t, 2H), 3.8 (s, 3H), 3.2(m, 4H), 2.7(m, 4H), 2.5(t, 2H), 1.8(m, 4H)

**Example 3**

**1-(2-methoxy-phenyl)-4-[4-(4-tetrazol-1-ylmethyl-phenoxy)-butyl]-piperazine.**

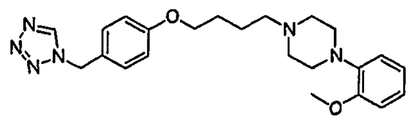

The procedure given in Example 1 was followed using 1H-tetrazole as reactants, instead of imidazole, to give 1-(2-methoxy-phenyl)-4-[4-(4-tetrazol-1-ylmethyl-phenoxy)-butyl]-piperazine.

$^1$H-NMR ($CDCl_3$, 200MHz) δ 8.6(s, 1H), 7.2 (m, 2H), 7.0(m, 6H), 5.6 (s, 2H), 4.0(t, 2H), 3.8(s, 3H)3.2(m, 4H), 2.9(m, 4H), 2.7(t, 2H), 1.9(m, 4H)

**Example 4**

**1-(2-methoxy-phenyl)-4-[4-(4-tetrazol-2-ylmethyl-phenoxy)-butyl]-piperazine.**

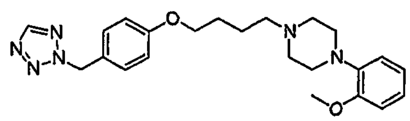

The procedure given in Example 1 was followed using 1H-tetrazole as reactants, instead of imidazole, to give 1-(2-methoxy-phenyl)-4-[4-(4-tetrazol-2-ylmethyl-phenoxy)-butyl]-piperazine.

$^1$H-NMR ($CDCl_3$, 200MHz) δ 8.6(s, 1H), 7.2 (m, 2H), 7.0(m, 6H), 5.8 (s, 2H), 4.0(t, 2H), 3.8(s, 3H)3.2(m, 4H), 2.9(m, 4H), 2.7(t, 2H), 1.9(m, 4H)

**Example 5**

**1-[4-(3-imidazol-1-ylmethyl-phenoxy)-butyl]-4-(2-methoxy-phenyl)-piperazine-hydrochloride.**

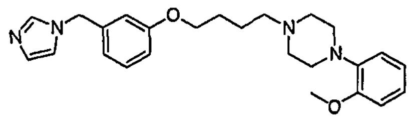

A mixture of 3-hydroxybenzaldehyde (5mmol), 1-bromo-4-chlorobutane (5mmol), and potassium carbonate (15mmol) was refluxed in 100ml of acetone for 6h. This solution was then concentrated in a rotary evaporator and diluted with ethyl acetate, then washed with brine, and the resulting organic layer was dried and purified by column chromatography. This was dissolved in methanol (50ml) and was added with sodium borohydride (10mmol) at 25°C, and was stirred at 25 °C for 2h. This solution was then concentrated in a rotary evaporator and diluted with methylene chloride, then washed with brine, and the resulting organic layer was dried and concentrated in vacuo. The crude product was dissolved in isopropanol (50ml) and was added with 1-(2-methoxyphenyl)-piperazine (5mmol), sodium carbonate (15mmol), and potassium iodide (5mmol) at 25°C, and the reaction mixture was refluxed for 12h. This solution was then concentrated in a rotary evaporator and diluted with methylene chloride, then washed with brine, and the resulting organic layer was dried and purified by column chromatography. This product was dissolved in $CHCl_3$ (30ml) and was added with pyridine and thionyl chloride (5mmol) at 0°C, and then the resulting reaction mixture was stirred at room temperature for 16h, and water was added to terminate the reaction. The organic layer was extracted 3 times with dichloromethane, then dried and concentrated in vacuo, and the residue was purified by column chromatography. This was dissolved in acetonitrile (30ml) and was added with imidazole (6mmol), potassium carbonate (15mmol), and potassium iodide (5mmol) at 25°C, and the reaction mixture was refluxed for 12h. This solution was then concentrated in a rotary evaporator and diluted with methylene chloride, then washed with brine, and the resulting organic layer was dried and purified by column chromatography. The resulting 1-[4-(3-imidazole-1-ylmethyl-phenoxy)-butyl]-4-(2-methoxy-phenyl)-piperazine was dissolved in dichloromethane and the solution was treated with a solution of HCl in ethyl ether. The resulting precipitate was filtered to give 1-[4-(3-imidazol-1-ylmethyl-phenoxy)-butyl]-4-(2-methoxy-phenyl)-piperazine hydrochloride.

$^1$H-NMR (DMSO, 200MHz) δ 11.6 (br,1H), 9.5 (s,1H),7.8 (s, 1H), 7.6 (s, 1H), 7.2(m, 2H), 7.0(m, 6H), 5.4(s, 2H), 4.0(t, 2H), 3.8(s, 3H), 3.6-3.2 (m, 10H), 2.0(m, 2H), 1.8(m, 2H)

**Example 6**

**1-(2-methoxy-phenyl)-4-[4-(3-[1,2,4]triazol-1-ylmethyl-phenoxy)-butyl]-piperazine hydrochloride.**

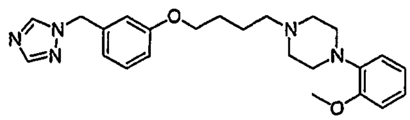

The procedure given in Example 5 was followed using 1H-1,2,4-triazole as reactants, instead of imidazole, to give 1-(2-methoxy-phenyl)-4-[4-(3-[1,2,4]triazol-1-ylmethyl-phenoxy)-butyl]-piperazine.

$^1$H-NMR (DMSO, 200MHz) δ 11.6 (br,1H), 9.5 (s,1H), 8.5(s, 1H), 7.2(m, 2H), 7.0(m, 6H), 5.4(s, 2H), 4.0(t, 2H), 3.8(s, 3H), 3.6-3.2 (m, 10H), 2.0(m, 2H), 1.8(m, 2H)

**Example 7**

**1-(2-methoxy-phenyl)-4-[4-(3-tetrazol-1-ylmethyl-phenoxy)-butyl]-piperazine hydrochloride.**

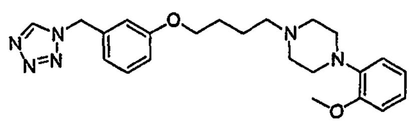

The procedure given in Example 5 was followed using 1H-tetrazole as reactants, instead of imidazole, to give 1-(2-methoxy-phenyl)-4-[4-(3-tetrazol-1-ylmethyl-phenoxy)-butyl]-piperazine.

$^1$H-NMR (DMSO, 200MHz) δ 10.8 (br,1H), 9.6 (s,1H), 7.2(m, 2H), 7.0(m, 6H), 5.4(s, 2H), 4.0(t, 2H), 3.8(s, 3H), 3.6 (m, 4H), 3.2 (m, 6H), 2.0(m, 2H), 1.8(m, 2H)

**Example 8**

**1-(2-methoxy-phenyl)-4-[4-(3-tetrazol-2-ylmethyl-phenoxy)-butyl]-piperazine hydrochloride.**

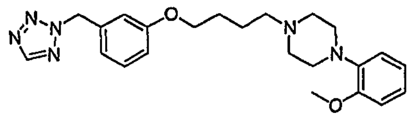

The procedure given in Example 5 was followed using 1H-tetrazole as reactants, instead of imidazole, to give 1-(2-methoxy-phenyl)-4-[4-(3-tetrazol-2-ylmethyl-phenoxy)-butyl]-piperazine.

$^1$H-NMR (DMSO, 200MHz) δ 10.8 (br,1H), 9.0 (s,1H), 7.2(m, 2H), 7.0(m, 6H), 5.9(s, 2H), 4.0(t, 2H), 3.8(s, 3H), 3.6 (m, 4H), 3.2 (m, 6H), 2.0(m, 2H), 1.8(m, 2H)

**Example 9**

**1-(4-{4-[4-(2-methoxy-phenyl)-piperazin-1-yl]-butoxy}-benzyl)-1H-benzoimidazole.**

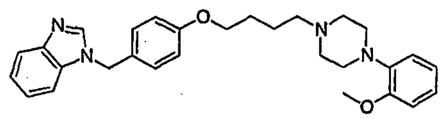

The procedure given in Example 1 was followed using 1H-benzoimidazole as reactants, instead of imidazole, to give 1-(4-{4-[4-(2-methoxy-phenyl)-piperazin-1-yl]-butoxy}-benzyl)-1 H-benzoimidazole.

$^1$H-NMR ($CDCl_3$, 200MHz) δ 8.0 (s, 1H), 7.8 (m, 1H), 7.3 (m, 3H), 7.2(m., 2H), 7.0(m, 6H), 5.3 (s, 2H), 4.0 (t, 2H), 3.8(s, 3H), 3.1(m, 4H), 2.7(m, 4H), 2.5(t, 2H), 1.8(m, 4H)

**Example 10**

**1-(2-Methoxy-phenyl)-4-{4-[4-(2-methyl-imidazol-1-ylmethyl)-phenoxy]-butyl}-piperazine.**

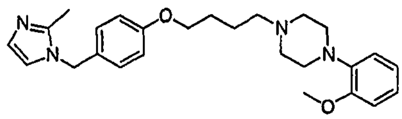

The procedure given in Example 1 was followed using 2-methyl-imidazole as reactants, instead of imidazole, to give 1-(2-Methoxy-phenyl)-4-{4-[4-(2-methyl-imidazol-1-ylmethyl)-phenoxy]-butyl}-piperazine.

$^1$H-NMR ($CDCl_3$, 200MHz) δ 7.1~6.8 (m, 10H), 5.0 (s, 2H), 4.0(t, 2H), 3.8 (s, 3H), 3.2(m, 4H), 2.7(m, 4H), 2.5(t, 2H), 2.2 (s,3H), 1.8(m, 4H)

**Example 11**

**1-(2-methoxy-phenyl)-4-[4-(4-[1,2,3]triazol-2-ylmethyl-phenoxy)-butyl]-piperazine**

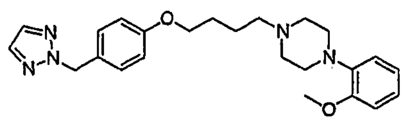

The procedure given in Example 1 was followed using 1H-1,2,3-triazole as reactants, instead of imidazole, to give 1-(2-methoxy-phenyl)-4-[4-(4-[1,2,3]triazole-2-ylmethyl-phenoxy)-butyl]-piperazine.

$^1$H-NMR ($CDCl_3$ 200MHz) δ 7.6 (s, 2H), 7.2(m, 2H), 6.9(m, 6H), 5.6 (s, 2H), 4.0(t, 2H), 3.8 (s, 3H), 3.2(m, 4H), 2.7(m, 4H), 2.5(t, 2H), 1.8(m, 4H)

**Example 12**

**1-(2-methoxy-phenyl)-4-[4-(4-[1,2,3]triazol-1-ylmethyl-phenoxy)-butyl]-piperazine**

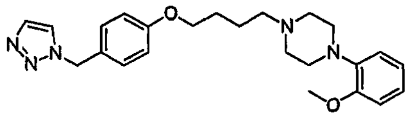

The procedure given in Example 1 was followed using 1H-1,2,3-triazole as reactants, instead of imidazole, to give 1-(2-methoxy-phenyl)-4-[4-(4-[1,2,3]triazole-1-ylmethyl-phenoxy)-butyl]-piperazine.

$^1$H-NMR ($CDCl_3$, 200MHz) δ 7.7 (s, 1H), 7.4 (s, 1H), 7.2(m, 2H), 6.9(m, 6H), 5.5 (s, 2H), 4.0(t, 2H), 3.8 (s, 3H), 3.2(m, 4H), 2.7(m, 4H), 2.5(t, 2H), 1.8(m, 4H)

**Example 13**

**1-(4-{4-[4-(2-Methoxy-phenyl)-piperazin-1-yl]-butoxy}-benzyl)-1H-indazole**

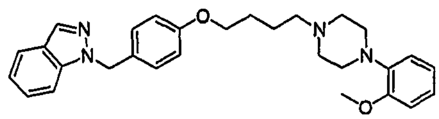

The procedure given in Example 1 was followed using 1H-Indazole as reactants, instead of imidazole, to give 1-(4-{4-[4-(2-methoxy-phenyl)-piperazin-1-yl]-butoxy}-benzyl)-1 H-indazole.

$^1$H-NMR ($CDCl_3$, 200MHz) δ 8.1 (s, 1H), 7.7 (m, 1H), 7.3 (m, 3H), 7.2(m., 2H), 7.0(m, 6H), 5.3 (s, 2H), 4.0 (t, 2H), 3.8(s, 3H), 3.1(m, 4H), 2.7(m, 4H), 2.5(t, 2H), 1.8(m, 4H)

**Example 14**

**1-(4-{4-[4-(2-Methoxy-phenyl)-piperazin-1-yl]-butoxy}-benzyl)-1H-benzotriazole.**

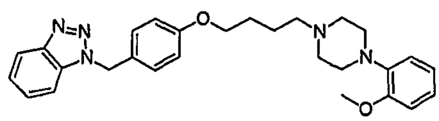

The procedure given in Example 1 was followed using benzotriazole as reactants, instead of imidazole, to give 1-(4-{4-[4-(2-Methoxy-phenyl)-piperazin-1-yl]-butoxy} -benzyl)-1 H-benzotriazole.

$^1$H NMR ($CDCl_3$, 200MHz) δ 8.1 (m, 1H), 7.4~7.2 (m, 5H), 7.0~6.8 (m, 6H), 5.8 (s, 2H), 4.0 (t, 2H), 3.8(s, 3H), 3.1(m, 4H), 2.7(m, 4H), 2.5(t, 2H), 1.8(m, 4H)

**Example 15**

**1-(2-methoxy-phenyl)-4-{4-[4-(5-phenyl-tetrazol-2-ylmethyl)-phenoxy]-butyl}-piperazine.**

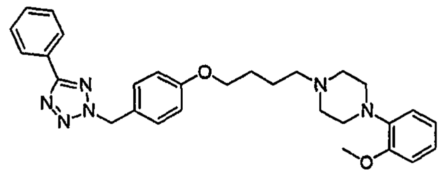

The procedure given in Example 1 was followed using 5-phenyl-1H-tetrazole as reactants, instead of imidazole, to give 1-(2-methoxy-phenyl)-4-{4-[4-(5-phenyl-tetrazol-2-ylmethyl)-phenoxy]-butyl}-piperazine.

$^1$H-NMR ($CDCl_3$, 200MHz) δ 8.2 (m, 2H), 7.6 (m, 5H), 7.2~7.0(m., 6H), 5.8 (s, 2H), 4.0 (t, 2H), 3.8(s, 3H), 3.1(m, 4H), 2.7(m, 4H), 2.5(t, 2H), 1.8(m, 4H)

**Example 16**

**1-(2-methoxy-phenyl)-4-{4-[4-(5-phenyl-tetrazol-1-ylmethyl)-phenoxy]-butyl}-piperazine**

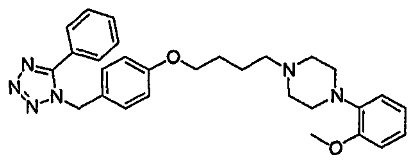

The procedure given in Example 1 was followed using 5-phenyl-1H-tetrazole as reactants, instead of imidazole, to give 1-(2-methoxy-phenyl)-4-{4-[4-(5-phenyl-tetrazol-1-ylmethyl)-phenoxy]-butyl}-piperazine.

$^1$H-NMR ($CDCl_3$, 200MHz) δ 7.6 (m, 4H), 7.2(m., 3H), 6.9 (m, 6H), 5.6 (s, 2H), 4.0 (t, 2H), 3.8(s, 3H), 3.1(m, 4H), 2.7(m, 4H), 2.5(t, 2H), 1.8(m, 4H)

**Example 17**

**1-(2-methoxy-phenyl)-4-{4-[4-(5-methyl-tetrazol-2-ylmethyl)-phenoxy]-butyl}-piperazine**

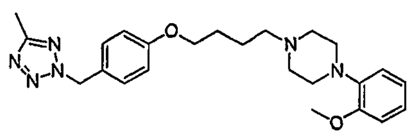

The procedure given in Example 1 was followed using 5-methyl-1H-tetrazole as reactants, instead of imidazole, to give 1-(2-methoxy-phenyl)-4-{4-[4-(5-methyl-tetrazol-2-ylmethyl)-phenoxy]-butyl}-piperazine.

$^1$H-NMR ($CDCl_3$, 200MHz) δ 7.4(m, 2H), 7.0(m, 6H), 5.6 (s, 2H), 4.0(t, 2H), 3.8(s, 3H), 3.1(m, 4H), 2.7(m, 4H), 2.5(t, 2H), 2.5(s, 3H), 1.8(m, 4H)

**Example 18**

**1-(2-methoxy-phenyl)-4-{4-[4-(5-methyl-tetrazol-1-ylmethyl)-phenoxy]-butyl}-piperazine**

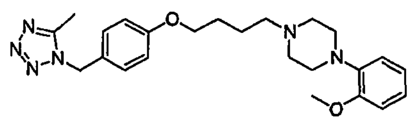

The procedure given in Example 1 was followed using 5-methyl-1H-tetrazole as reactants, instead of imidazole,

to give 1-(2-methoxy-phenyl)-4-{4-[4-(5-methyl-tetrazol-1-ylmethyl)-phenoxy]-butyl}-piperazine.

$^1$H NMR ($CDCl_3$, 200MHz) δ 7.2 (m, 2H), 7.0(m, 6H), 5.4 (s, 2H), 4.0(t, 2H), 3.8(s, 3H), 3.1(m, 4H), 2.7(m, 4H), 2.5(t, 2H), 2.5(s, 3H), 1.8(m, 4H)

**Example 19**

**(4-{4-[4-(2-methoxy-phenyl)-piperazin-1-yl]-butoxy}-benzyl)-dimethyl-amine dihydrochloride**

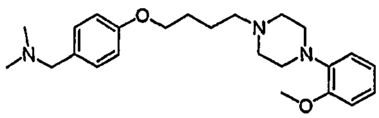

The procedure given in Example 1 was followed using dimethyl amine as reactants, instead of imidazole, to give (4-{4-[4-(2-methoxy-phenyl)-piperazin-1-yl]-butoxy}-benzyl)-dimethyl-amine dihydrochloride.

$^1$H-NMR (DMSO, 200MHz) δ 11.3 (br,1H), 11.1 (br,1H), 7.5(m, 2H), 7.0(m, 6H), 4.2 (m, 2H), 4.0(t, 2H), 3.8(s, 3H), 3.6 (m, 4H), 3.2 (m, 6H), 2.6(m, 6H), 1.8(m, 4H)

**Example 20**

**1-(2-methoxy-phenyl)-4-[4-(4-piperidin-1-ylmethyl-phenoxy)-butyl]-piperazine**

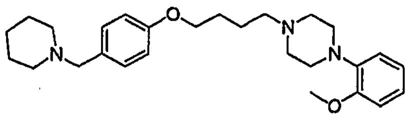

The procedure given in Example 1 was followed using piperidine as reactants, instead of imidazole, to give 1-(2-methoxy-phenyl)-4-[4-(4-piperidin-1-ylmethyl-phenoxy)-butyl]-piperazine.

$^1$H-NMR ($CDCl_3$, 200MHz) δ 7.2(m, 2H), 6.9(m, 6H), 4.0(t, 2H), 3.8 (s, 3H), 3.4 (s, 2H), 3.2(m, 4H), 2.7(m, 4H), 2.5(t, 2H), 2.4 (m,4H), 1.8(m, 4H), 1.6(m, 4H), 1.4 (m,2H)

**Example 21**

**(4-{4-[4-(2-methoxy-phenyl)-piperazin-1-yl]-butoxy}-benzyl)-phenyl-amine**

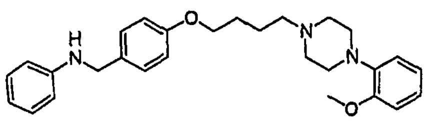

The procedure given in Example 1 was followed using aniline as reactants, instead of imidazole, to give (4-{4-[4-(2-methoxy-phenyl)-piperazin-1-yl]-butoxy}-benzyl)-phenyl-amine.

$^1$H-NMR ($CDCl_3$, 200MHz) δ 7.2(m, 2H), 7.0(m, 6H), 6.8 (m, 5H), 4.3(s, 2H), 4.0(t, 2H), 3.8 (s, 3H), 3.6 (s, 1H), 3.2(m, 4H), 2.7(m, 4H), 2.5(t, 2H), 1.8(m, 4H)

**Example 22 (10663)**

**(4-{4-[4-(2-methoxy-phenyl)-piperazin-1-yl]-butoxy}-benzyl)-thiazol-2-yl-amine**

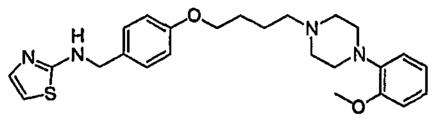

The procedure given in Example 1 was followed using thiazol-2-ylamine as reactants, instead of imidazole, to give (4-{4-[4-(2-methoxy-phenyl)-piperazin-1-yl]-butoxy} -benzyl)-thiazol-2-yl-amine.

$^1$H-NMR ($CDCl_3$, 200MHz) δ 7.5 (s, 1H), 7.2(m, 2H), 7.0(m, 6H), 6.6 (s, 1H), 4.3(s, 2H), 4.0(t, 2H), 3.8 (s, 3H), 3.6 (s, 1H), 3.2(m, 4H), 2.7(m, 4H), 2.5(t, 2H), 1.8(m, 4H)

**Example 23**

**1-(2-chloro-phenyl)-4-[4-(4-[1,2,4]triazol-1-ylmethyl-phenoxy)-butyl]-piperazine**

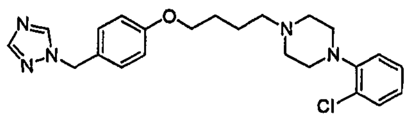

The procedure given in Example 1 was followed using 1-(2-chloro-phenyl)-piperazine and 1H-1,2,4-triazole as reactants, instead of 1-(2-methoxyphenyl)-piperazine and imidazole, to give 1-(2-chloro-phenyl)-4-[4-(4-[1,2,4]triazol-1-ylmethyl-phenoxy)-butyl]-piperazine.

$^1$H-NMR ($CDCl_3$, 200MHz) δ 8.1 (s, 1H), 8.0 (s, 1H), 7.2(m, 2H), 6.9(m, 6H), 5.2(s, 2H), 4.0(t, 2H), 3.2(m, 4H), 2.7(m, 4H), 2.5(t, 2H), 1.8(m, 4H)

**Example 24**

**1-(2-methoxy-phenyl)-4-[4-(3-[1,2,3]triazol-2-ylmethyl-phenoxy)-butyl]-piperazine**

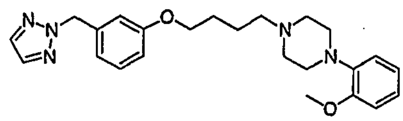

The procedure given in Example 5 was followed using 1H-1,2,3-triazole as reactants, instead of imidazole, to give 1-(2-methoxy-phenyl)-4-[4-(3-[1,2,3]triazol-2-ylmethyl-phenoxy)-butyl]-piperazine.

$^1$H-NMR ($CDCl_3$, 200MHz) δ 7.6 (s, 2H), 7.2(m, 2H), 6.9(m, 6H), 5.4 (s, 2H), 4.0(t, 2H), 3.8 (s, 3H), 3.2(m, 4H), 2.7(m, 4H), 2.5(t, 2H), 1.8(m, 4H)

**Example 25**

**1-(2-Methoxy-phenyl)-4-[4-(3-[1,2,3]triazol-1-ylmethyl-phenoxy)-butyl]-piperazine.**

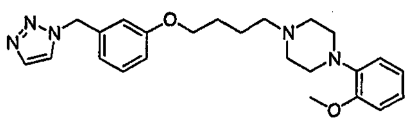

The procedure given in Example 5 was followed using 1H-1,2,3-triazole as reactants, instead of Imidazole, to give 1-(2-Methoxy-phenyl)-4-[4-(3-[1,2,3]triazol-1-ylmethyl-phenoxy)-butyl]-piperazine.

$^1$H-NMR ($CDCl_3$, 200MHz) δ 7.7 (s, 1H), 7.4 (s, 1H), 7.2 (m, 2H), 7.0-6.8 (m, 6H), 5.5 (s, 2H), 4.0(t, 2H), 3.8(s, 3H), 3.1(m, 4H), 2.7(m, 4H), 2.5(t, 2H), 2.5(s, 3H),1.8(m, 4H)

**Example 26**

**1-(2-Methoxy-phenyl)-4-{4-[3-(5-methyl-tetrazol-2-ylmethyl)-phenoxy]-butyl}-piperazine.**

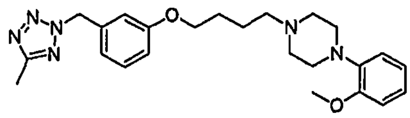

The procedure given in Example 5 was followed using 5-methyl-1H-tetrazole as reactants, instead of imidazole, to give 1-(2-Methoxy-phenyl)-4-{4-[3-(5-methyltetrazol-2-ylmethyl)-phenoxy]-butyl}-piperazine.

$^1$H-NMR ($CDCl_3$, 200MHz) δ 7.6 (s, 2H), 7.2 (m, 2H), 7.0-6.8 (m, 6H), 5.5 (s, 2H), 4.0(t, 2H), 3.8(s, 3H), 3.1(m, 4H), 2.7(m, 4H), 2.5(t, 2H), 2.5(s, 3H),1.8(m, 4H)

**Example 27**

**1-(2-methoxy-phenyl)-4-{4-[3-(5-methyl-tetrazol-1-ylmethyl)-phenoxy]-butyl}-piperazine**

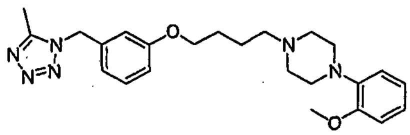

The procedure given in Example 5 was followed using 5-methyl-1H-tetrazole as reactants, instead of imidazole, to give 1-(2-methoxy-phenyl)-4-{4-[3-(5-methyl-tetrazol-1-ylmethyl)-phenoxy]-butyl}-piperazine.

$^1$H-NMR ($CDCl_3$, 200MHz) δ 7.2 (m, 2H), 7.0-6.7 (m, 6H), 5.5 (s, 2H), 4.0(t, 2H), 3.8(s, 3H), 3.1(m, 4H), 2.7(m, 4H), 2.5(t, 2H), 2.5(s, 3H),1.8(m, 4H)

**Example 28**

**(3-{4-[4-(2-methoxy-phenyl)-piperazin-1-yl]-butoxy}-benzyl)-dimethyl-amine**

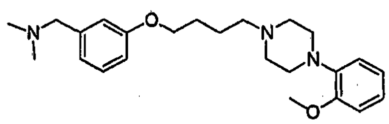

The procedure given in Example 5 was followed using dimethyl amine as reactants, instead of imidazole, to give (3-{4-[4-(2-methoxy-phenyl)-piperazin-1-yl]-butoxy}-benzyl)-dimethyl-amine.

$^1$H-NMR ($CDCl_3$, 200MHz) δ 7.3(m, 2H), 6.9(m, 6H), 4.0(t, 2H), 3.9(s, 3H), 3.2(m, 4H), 2.7(m, 4H), 2.6(s, 6H), 2.5(t, 2H), 1.8(m, 4H)

**Example 29**

**4-(3-{4-[4-(2-methoxy-phenyl)-piperazin-1-yl]-butoxy}-benzyl)-morpholine**

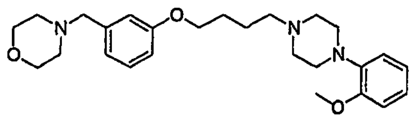

The procedure given in Example 5 was followed using morpholine as reactants, instead of imidazole, to give 4-(3-{4-[4-(2-methoxy-phenyl)-piperazin-1-yl]-butoxy}-benzyl)-morpholine.

$^1$H-NMR ($CDCl_3$, 200MHz) 7.2(m, 2H), 6.9(m, 6H), 4.1(t, 2H), 3.9(s, 3H), 3.7(m,4H), 3.5 (s, 2H), 3.2(m, 4H), 2.8(m, 4H), 2.6(t, 2H), 2.5 (m,4H), 1.8(m, 4H)

**Example 30**

**1-{4-[4-(4,5-dichloro-imidazol-1-ylmethyl)-phenoxy]-butyl}-4-(2-methoxy-phenyl)-piperazine**

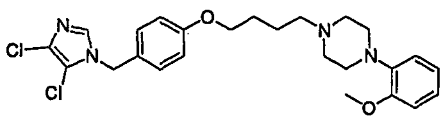

The procedure given in Example 1 was followed using 4,5-dichloro-imidazole as reactants, instead of imidazole, to give 1-{4-[4-(4,5-dichloro-imidazol-1-ylmethyl)-phenoxy]-butyl} -4-(2-methoxy-phenyl)-piperazine.

$^1$H-NMR ($CDCl_3$, 200MHz) δ 7.4 (s. 1 H), 7.2(m, 2H), 6.9(m, 6H), 5.0 (s, 2H), 4.0(t, 2H), 3.8 (s, 3H), 3.2(m, 4H), 2.7(m, 4H), 2.5(t, 2H), 1.8(m, 4H)

**Example 31**

**1-(2-chloro-phenyl)-4-[4-(4-tetrazol-2-ylmethyl-phenoxy)-butyl]-piperazine**

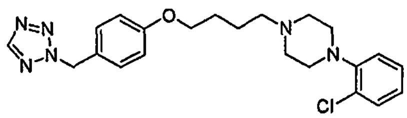

The procedure given in Example 1 was followed using 1-(2-chloro-phenyl)-piperazine and 1H-tetrazole as reactants, instead of 1-(2-methoxyphenyl)-piperazine and imidazole, to give 1-(2-chloro-phenyl)-4-[4-(4-tetrazol-2-ylmethyl-phenoxy)-butyl]-piperazine.

$^1$H-NMR ($CDCl_3$, 200MHz) δ 8.5(m, 1H), 7.4(m, 4H), 7.0(m, 2H), 6.8(m, 2H), 5.8 (s, 2H), 4.0(t, 2H), 3.2(m, 4H), 2.9(m, 4H), 2.7(t, 2H), 1.9(m, 4H)

**Example 32**

**4-(4-{4-[4-(2-methoxy-phenyl)-piperazin-1-yl]-butoxy}-benzyl)-morpholine**

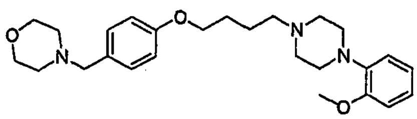

The procedure given in Example 1 was followed using morpholine as reactants, instead of imidazole, to give 4-(4-{4-[4-(2-methoxy-phenyl)-piperazin-1-yl]-butoxy}-benzyl)-morpholine.

$^1$H-NMR ($CDCl_3$, 200MHz) δ 7.2(m, 2H), 6.9(m, 6H), 4.0(t, 2H), 3.8 (s, 3H), 3.4 (s, 2H), 3.2(m, 4H), 2.7(m, 4H), 2.5(t, 2H), 2.4 (m,4H), 1.8(m, 4H), 1.6(m, 4H)

**Example 33**

**(4-{4-[4-(2-Chloro-phenyl)-piperazin-1-yl]-butoxy}-benzyl)-dimethyl-amine**

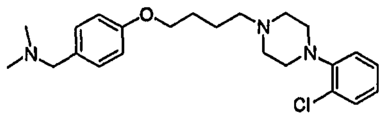

The procedure given in Example 1 was followed using 1-(2-chloro-phenyl)-piperazine and dimethylamine, instead of 1-(2-methoxyphenyl)-piperazine and imidazole, to give (4-{4-[4-(2-Chloro-phenyl)-piperazin-1-yl]-butoxy}-benzyl)-dimethyl-amine.

$^1$H-NMR ($CDCl_3$, 200MHz) δ 7.3~7.1 (m, 4H), 7.1~6.8 (m, 4H), 4.2 (t, 2H), 3.4(m, 2H), 3.1(m, 4H), 2.7 (m, 4H), 2.5 (m,2H), 2.4 (s,6H), 1.8~1.7 (m, 4H)

**Example 34**

**1-(3-fluoro-phenyl)-4-[4-(4-[1,2,4]triazol-1-ylmethyl-phenoxy)-butyl]-piperazine**

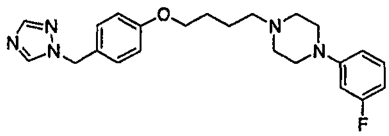

The procedure given in Example 1 was followed using 1-(3-fluoro-phenyl)-piperazine and 1H-1,2,4-triazole as reactants, instead of 1-(2-methoxyphenyl)-piperazine and imidazole, to give 1-(3-fluoro-phenyl)-4-[4-(4-[1,2,4]triazol-1-ylmethyl-phenoxy)-butyl]-piperazine.

$^1$H-NMR ($CDCl_3$, 200MHz) δ 7.9 (s, 1H), 7.7 (s, 1H), 7.2(m, 2H), 6.9(m, 6H), 5.5(s, 2H), 4.0(t, 2H), 3.2(m, 4H), 2.8(m, 4H), 2.6(t, 2H), 1.8(m, 4H)

**Example 35**

**1-(2-methoxy-phenyl)-4-[4-(4-4-methylpiperazine-1-ylmethyl-phenoxy)-butyl]-piperazine**

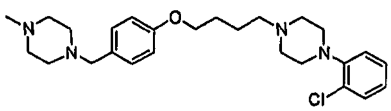

The procedure given in Example 1 was followed using 1-(2-chloro-phenyl)-piperazine and methylpiperazine, instead of instead of 1-(2-methoxyphenyl)-piperazine and imidazole, to give 1-(2-methoxy-phenyl)-4-[4-(4-4-methylpiperazine-1-ylmethyl-phenoxy)-butyl]-piperazine.

$^1$H-NMR ($CDCl_3$, 200MHz) δ 7.3~7.1 (m, 4H), 7.1~6.8 (m, 4H), 4.3 (t, 2H), 3.4(m, 2H), 3.1(m, 4H), 2.7 (m, 4H), 2.6 (m, 8H,) 2.5 (m, 2H), 2.3 (s, 3H), 1.8~1.7 (m, 4H)

**Example 36**

**1-(2,3-dichloro-phenyl)-4-[4-(4-tetrazol-2-ylmethyl-phenoxy)-butyl]-piperazine**

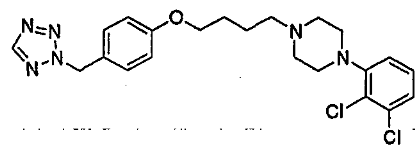

The procedure given in Example 1 was followed using 1-(2,3-dichloro-phenyl)-piperazine and 1H-tetrazole as reactants, instead of 1-(2-methoxyphenyl)-piperazine and imidazole, to give 1-(2,3-dichloro-phenyl)-4-[4-(4-tetrazol-2-ylmethyl-phenoxy)-butyl]-piperazine.

$^1$H-NMR ($CDCl_3$, 200MHz) δ 8.5 (m, 1H), 7.4-6.8 (m, 7H), 5.8 (s, 2H), 4.0(t, 2H), 3.2(m, 4H), 2.9(m, 4H), 2.7(t, 2H), 1.9(m, 4H)

**Example 37**

**1-(2,3-Dichloro-phenyl)-4-{4-[4-(5-methyl-tetrazol-1-ylmethyl)-phenoxy]-butyl}-piperazine**

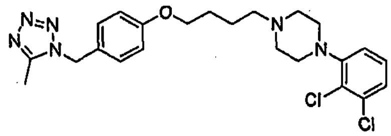

The procedure given in Example 1 was followed using 1-(2,3-dichloro-phenyl)-piperazine and 5-methyl-1H-tetrazole as reactants, instead of 1-(2-methoxyphenyl)-piperazine and imidazole, to give 1-(2,3-Dichloro-phenyl)-4-{4-[4-(5-methyl-tetrazol-1-ylmethyl)-phenoxy]-butyl}-piperazine.

$^1$H-NMR ($CDCl_3$, 200MHz) δ 7.2 (m, 2H), 7.0(m, 5H), 5.4 (s, 2H), 4.0(t, 2H), 3.1(m, 4H), 2.7(m, 4H), 2.5(t, 2H), 2.6(s, 3H), 1.8(m, 4H)

**Example 38**

**1-(2,3-Dichloro-phenyl)-4-{4-[4-(5-methyl-tetrazol-2-ylmethyl)-phenoxy]-butyl}-piperazine**

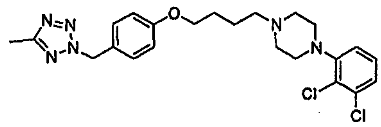

The procedure given in Example 1 was followed using 1-(2,3-dichloro-phenyl)-piperazine and 5-methyl-1H-tetrazole as reactants, instead of 1-(2-methoxyphenyl)-piperazine and imidazole, to give 1-(2,3-Dichloro-phenyl)-4-{4-[4-(5-methyl-tetrazol-2-ylmethyl)-phenoxy]-butyl}-piperazine.

$^1$H-NMR ($CDCl_3$, 200MHz) δ 7.2~7.0(m, 7H), 5.6 (s, 2H), 4.0(t, 2H), 3.1(m, 4H), 2.7(m, 4H), 2.5(t, 2H), 2.5(s, 3H), 1.8(m, 4H)

**Example 39**

**(4-{4-[4-(2,3-dichloro-phenyl)-piperazin-1-yl]-butoxy}-benzyl)-dimethyl-amine**

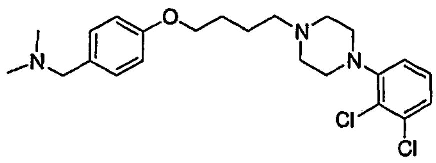

The procedure given in Example 1 was followed using 1-(2,3-dichloro-phenyl)-piperazine and dimethyl amine as reactants, instead of 1-(2-methoxyphenyl)-piperazine and imidazole, to give (4-{4-[4-(2,3-dichloro-phenyl)-piperazin-1-yl]-butoxy}-benzyl)-dimethyl-amine.

$^{1}$H-NMR (DMSO, 200MHz) $\delta$ 11.3 (br,1H), 11.1 (br,1H), 7.5(m, 2H), 7.0(m, 5H), 4.2 (m, 2H), 4.0(t, 2H), 3.6 (m, 4H), 3.2 (m, 6H), 2.6(m, 6H), 1.8(m, 4H)

**Example 40**

**4-(4-{4-[4-(2,3-Dichloro-phenyl)-piperazin-1-yl]-butoxy}-benzyl)-morpholine**

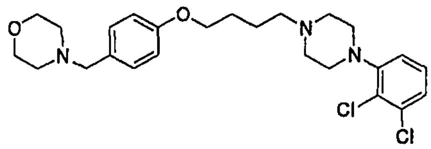

The procedure given in Example 1 was followed using 1-(2,3-dichloro-phenyl)-piperazine and morpholine, instead of 1-(2-methoxyphenyl)-piperazine and imidazole, to give 4-(4-{4-[4-(2,3-Dichloro-phenyl)-piperazin-1-yl]-butoxy}-benzyl)-morpholine

$^{1}$H-NMR ($CDCl_3$, 200MHz) $\delta$ 7.2(m, 2H), 6.9(m, 6H), 4.2(t, 2H), 3.7(m,4H), 3.5 (s, 2H), 3.2(m, 4H), 2.8(m, 4H), 2.6(t, 2H), 2.5 (m,4H), 1.8(m, 4H)

**Example 41**

**1-(2-methoxy-phenyl)-4-[4-(4-4-methylpiperazine-1-ylmethyl-phenoxy)-butyl]-piperazine**

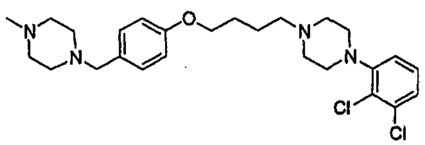

The procedure given in Example 1 was followed using 1-(2,3-dichloro-phenyl)-piperazine and methylpiperazine, instead ofl-(2-methoxyphenyl)-piperazine and imidazole to give 1-(2-methoxy-phenyl)-4-[4-(4-4-methylpiperazine-1-ylmethyl-phenoxy)-butyl]-piperazine.

$^{1}$H-NMR ($CDCl_3$, 200MHz) $\delta$ 7.3~6.8 (m, 7H), 4.3 (t, 2H), 3.4(m, 2H), 3.1 (m, 4H), 2.7 (m, 4H), 2.6 (m, 8H,) 2.5 (m, 2H), 2.3 (s, 3H), 1.8~1.7 (m, 4H)

**Example 42**

**1-(2-chloro-phenyl)-4-[4-(4-[1,2,3]triazol-1-ylmethyl-phenoxy)-butyl]-piperazine**

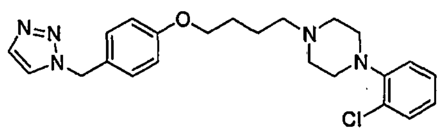

The procedure given in Example 1 was followed using 1-(2-chloro-phenyl)-piperazine and 1H-1,2,3-triazole as reactants, instead of 1-(2-methoxyphenyl)-piperazine and imidazole, to give 1-(2-chloro-phenyl)-4-[4-(4-[1,2,3]triazol-1-ylmethyl-phenoxy)-butyl]-piperazine.

$^1$H-NMR ($CDCl_3$, 200MHz) $\delta$ 7.7 (s, 1H), 7.4 (s, 1H), 7.2(m, 2H), 6.9(m, 6H), 5.5(s, 2H), 4.0(t, 2H), 3.2(m, 4H), 2.7(m, 4H), 2.5(t, 2H), 1.8(m, 4H)

**Example 43**

**(4-{4-[4-(2-Methoxy-phenyl)-piperazin-1-yl]-butoxy}-benzyl)-[1,3,4]thiadiazol-2-ylamine**

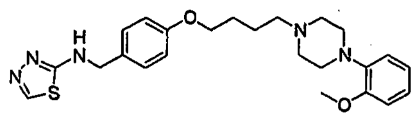

The procedure given in Example 1 was followed using [1,3,4]Thiadiazol-2-ylamine as reactants, instead of imidazole, to give (4-{4-[4-(2-Methoxy-phenyl)-piperazin-1-yl]-butoxy}-benzyl)-[1,3,4]thiadiazol-2-yl-amine.

$^1$H-NMR ($CDCl_3$, 200MHz) $\delta$ 7.2~ 6.8 (m, 9H), 4.3(s, 2H), 4.0(t, 2H), 3.8 (s, 3H), 3.2(m, 4H), 2.7(m, 4H), 2.5(t, 2H), 1.8(m, 4H)

**Example 44**

**1-(2,3-dichloro-phenyl)-4-[4-(4-[1,2,3]triazol-1-ylmethyl-phenoxy)-butyl]-piperazine**

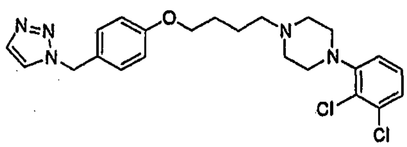

The procedure given in Example 1 was followed using 1-(2,3-dichloro-phenyl)-piperazine and 1H-1,2,3-triazole as reactants, instead of 1-(2-methoxyphenyl)-piperazine and imidazole, to give 1-(2,3-dichloro-phenyl)-4-[4-(4-[1,2,3]triazol-1-ylmethyl-phenoxy)-butyl]-piperazine.

$^1$H-NMR ($CDCl_3$, 200MHz) $\delta$ 7.7 (s, 1H), 7.4-6.8 (m, 8H), 5.8 (s, 2H), 4.0(t, 2H), 3.2(m, 4H), 2.9(m, 4H), 2.7(t, 2H), 1.9(m, 4H)

**Example 45**

**(4-{4-[4-(2-Methoxy-phenyl)-piperazin-1-yl]-butoxy}-benzyl)-(5-methyl-isoxazol-3-yl)-amine**

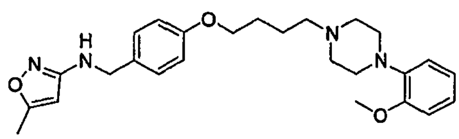

The procedure given in Example 1 was followed using 5-methyl-isoxazol-3-ylamine as reactants, instead of imidazole, to give (4-{4-[4-(2-Methoxy-phenyl)-piperazin-1-yl]-butoxy}-benzyl)-(5-methyl-isoxazol-3-yl)-amine.

$^1$H-NMR ($CDCl_3$, 200MHz) δ 7.2~6.8(m, 9H), 4.3(s, 2H), 4.0(t, 2H), 3.8 (s, 3H), 3.6 (s, 1H)3.2(m, 4H), 2.7(m, 4H), 2.5(t, 2H), 2.3 (s, 3H), 1.8(m, 4H)

**Example 46**

**(4-{4-[4-(2-methoxy-phenyl)-piperazin-1-yl]-butoxy}-benzyl)-(3-methyl-isoxazol-5-yl)-amine**

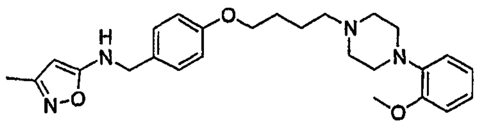

The procedure given in Example 1 was followed using 3-methyl-isoxazol-5-ylamine as reactants, instead of imidazole, to give (4-{4-[4-(2-methoxy-phenyl)-piperazin-1-yl]-butoxy}-benzyl)-(3-methyl-isoxazol-5-yl)-amine.

$^1$H-NMR ($CDCl_3$, 200MHz) δ 7.2(m, 2H), 7.0(m, 6H), 6.8 (s, 1H), 4.3(s, 2H), 4.0(t, 2H), 3.8 (s, 3H), 3.6 (s, 1H), 3.2(m, 4H), 2.7(m, 4H), 2.5(t, 2H), 2.3 (s,3H), 1.8(m, 4H)

**Example 47**

**1-{4-[4-(2-imidazol-1-yl-ethyl)-phenoxy]-butyl}-4-(2-methoxy-phenyl)-piperazine**

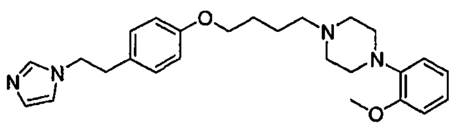

A mixture of 4-hydroxyphenetylalcohol (5mmol), 1-bromo-4-chlorobutane (5mmol), and potassium carbonate (15mmol) was refluxed in 100ml of acetone for 6h. This solution was then concentrated in a rotary evaporator and diluted with ethyl acetate. This mixture was then washed with brine, and the resulting organic layer was dried and purified by column chromatography. The product was dissolved in isopropanol (50ml) and was added with 1-(2-methoxyphenyl)-piperazine (5mmol), sodium carbonate (15mmol), and potassium iodide (5mmol) at 25 °C and the reaction mixture was refluxed for 12h. This solution was then concentrated in a rotary evaporator and diluted with methylene chloride. This mixture was then washed with brine, and the resulting organic layer was dried and purified by column chromatography. The product was dissolved in THF and was added with imidazole (15mmol), triphenylphosphine (15mmol), and diisopropyl azodicarboxylate (15mmol) dropwise at 0°C and warmed to room temperature. After 2h, the solvent was removed and the residue was washed with brine, and then purified by column chromatography.

$^1$H-NMR ($CDCl_3$, 200MHz) δ 7.3 (s, 1 H), 7.0(m, 10H), 4.1(t, 2H), 4.0(t, 2H), 3.8 (s, 3H), 3.1(m, 4H), 3.0(m, 2H), 2.7(m, 4H), 2.6(t, 2H), 1.8(m, 4H)

**Example 48**

**1-(2-methoxy-phenyl)-4-{4-[4-(2-[1,2,4]triazol-1-yl-ethyl)-phenoxy]-butyl}-piperazine**

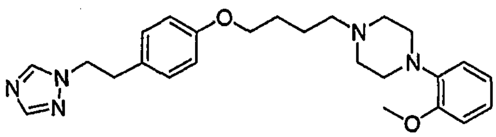

The procedure given in Example 47 was followed using 1H-1,2,4-triazole as a reactant, instead of imidazole,

to give 1-(2-methoxy-phenyl)-4-{4-[4-(2-[1,2,4]triazol-1-yl-ethyl)-phenoxy]-butyl}-piperazine.

$^1$H-NMR ($CDCl_3$, 200MHz) $\delta$ 8.0(s, 1H), 7.8(s, 1H), 7.0(m, 8H), 4.4(t, 2H), 4.0(t, 2H), 3.8 (s, 3H), 3.1(m, 6H), 2.7(m, 4H), 2.5(t, 2H), 1.8(m, 4H)

**Example 49**

**1-(2-methoxy-phenyl)-4-{4-[4-(2-[1,2,3]triazol-2-yl-ethyl)-phenoxy]-butyl}-piperazine**

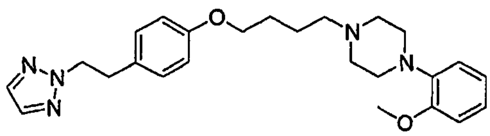

The procedure given in Example 47 was followed using 1H-1,2,3-triazole as a reactant, instead of imidazole, to give 1-(2-methoxy-phenyl)-4-{4-[4-(2-[1,2,3]triazol-2-yl-ethyl)-phenoxy]-butyl}-piperazine.

$^1$H-NMR ($CDCl_3$, 200MHz) $\delta$ 7.7(s, 2H), 7.0(m, 8H), 4.6(t, 2H), 4.0(t, 2H), 3.8 (s, 3H), 3.1(m, 6H), 2.7(m, 4H), 2.5(t, 2H), 1.8(m, 4H)

**Example 50**

**1-(2-methoxy-phenyl)-4-{4-[4-(2-[1,2,3]triazol-1-yl-ethyl)-phenoxy]-butyl}-piperazine**

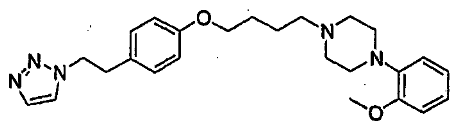

The procedure given in Example 47 was followed using 1H-1,2,3-triazole as a reactant, instead of imidazole, to give 1-(2-methoxy-phenyl)-4-{4-[4-(2-[1,2,3]triazol-1-yl-ethyl)-phenoxy]-butyl}-piperazine.

$^1$H-NMR ($CDCl_3$, 200MHz) $\delta$ 7.6(s, 1H), 7.3(s, 1H), 7.0(m, 8H), 4.6(t, 2H), 4.0(t, 2H), 3.8 (s, 3H), 3.1(m, 6H), 2.7(m, 4H), 2.5(t, 2H), 1.8(m, 4H)

**Example 51**

**1-(2-methoxy-phenyl)-4-{4-[4-(2-tetrazol-2-yl-ethyl)-phenoxy]-butyl}-piperazine**

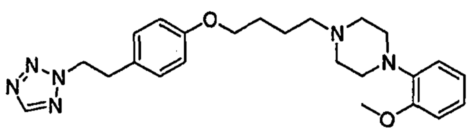

The procedure given in Example 47 was followed using 1H-tetrazole as a reactant, instead of imidazole, to give 1-(2-methoxy-phenyl)-4-{4-[4-(2-tetrazol-2-yl-ethyl)-phenoxy]-butyl}-piperazine.

$^{1}$H-NMR ($CDCl_3$, 200MHz) δ 8.5(s, 1H), 7.0(m, 8H), 4.9(t, 2H), 4.0(t, 2H), 3.8 (s, 3H), 3.3(t, 2H), 3.1(m, 4H), 2.7(m, 4H), 2.5(t, 2H), 1.8(m, 4H)

**Example 52**

**1-(2-methoxy-phenyl)-4-{4-[4-(2-tetrazol-1-yl-ethyl)-phenoxy]-butyl}-piperazine**

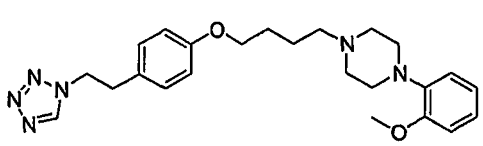

The procedure given in Example 47 was followed using 1H-tetrazole as a reactant, instead of imidazole, to give 1-(2-methoxy-phenyl)-4-{4-[4-(2-tetrazol-1-yl-ethyl)-phenoxy]-butyl}-piperazine.

$^{1}$H-NMR ($CDCl_3$, 200MHz) δ 8.2(s, 1H), 7.0(m, 8H), 4.7(t, 2H), 4.0(t, 2H), 3.8 (s, 3H), 3.1 (m, 6H), 2.7(m, 4H), 2.5(t, 2H), 1.8(m, 4H)

**Example 53**

**1-(2-Methoxy-phenyl)-4-(4-{4-[2-(5-methyl-tetrazol-1-yl)-ethyl]-phenoxy}-butyl)-piperazine**

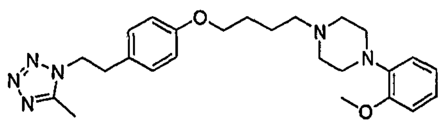

The procedure given in Example 47 was followed using 5-Methyl-tetrazole as a reactant, instead of imidazole, to give 1-(2-Methoxy-phenyl)-4-(4-{4-[2-(5-methyl-tetrazol-1-yl)-ethyl]-phenoxy} -butyl)-piperazine.

$^{1}$H-NMR ($CDCl_3$, 200MHz) δ 7.2~7.0(m, 8H), 5.0 (t, 2H), 4.0(t, 2H), 3.8 (s, 3H), 3.1(m, 6H), 2.7(m, 4H), 2.3~2.5 (m, 5H), 1.8(m, 4H)

**Example 54**

**1-(2-Methoxy-phenyl)-4-(4-{4-[2-(5-methyl-tetrazol-2-yl)-ethyl]-phenoxy}-butyl)-piperazine.**

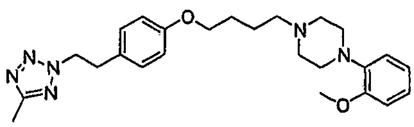

The procedure given in Example 47 was followed using 5-Methyl-tetrazole as a reactant, instead of imidazole, to give 1-(2-Methoxy-phenyl)-4-(4-{4-[2-(5-methyl-tetrazol-2-yl)-ethyl]-phenoxy}-butyl)-piperazine.

$^{1}$H-NMR ($CDCl_3$, 200MHz) δ 7.2~7.0(m, 8H), 5.2 (t, 2H), 4.0(t, 2H), 3.8 (s, 3H), 3.1(m, 6H), 2.7(m, 4H), 2.3~2.5 (m, 5H), 1.8(m, 4H)

**Example 55**

**2-{4-[4-(4-[1,2,3]-triazol-1-ylmethyl-phenoxy)-butyl]-piperazin-1-yl}-benzonitrile**

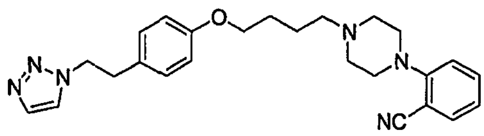

The procedure given in Example 1 was followed using 1-(2-cyano-phenyl)-piperazine and 1H-1,2,3-triazole as reactants, instead of 1-(2-methoxyphenyl)-piperazine and imidazole, to give 2-{4-[4-(4-[1,2,3]-triazol-1-ylmethyl-phenoxy)-butyl]-piperazin-1-yl}-benzonitrile.
$^1$H-NMR ($CDCl_3$, 200MHz) δ 7.9 (s, 1H), 7.8 (s, 1H), 7.2(m, 2H), 6.9(m, 6H), 5.5(s, 2H), 4.0(t, 2H), 3.2(m, 4H), 2.8(m, 4H), 2.6(t, 2H), 1.8(m, 4H)

**Example 56**

**1-(2-chloro-phenyl)-4-[4-(4-[1,2,3]triazol-2-ylmethyl-phenoxy)-butyl]-piperazine**

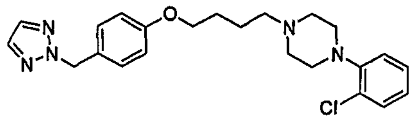

The procedure given in Example 1 was followed using 1-(2-chloro-phenyl)-piperazine and 1H-1,2,3-triazole as reactants, instead of 1-(2-methoxyphenyl)-piperazine and imidazole, to give 1-(2-chloro-phenyl)-4-[4-(4-[1,2,3]triazol-1-ylmethyl-phenoxy)-butyl]-piperazine.
$^1$H-NMR ($CDCl_3$, 200MHz) δ 7.6 (s, 2H), 7.2(m, 2H), 6.9(m, 6H), 5.6 (s, 2H), 4.0(t, 2H), 3.2(m, 4H), 2.7(m, 4H), 2.5(t, 2H), 1.8(m, 4H)
The therapeutic use of the compounds of general structural formula (I) and their pharmaceutically useful salts are demonstrated by the following tests.

**Example 57: Serotonin 1A (5-HT1A) receptor inhibition assay**

The method to test the ability of compounds to inhibit serotonin 1A(5-HT1A) receptors was using Hall. et al., J NeuroChem., 1985, 44, p1685~1696 with modification.
The Sprague-Dawley (SD) rats were killed by decapitation and their brains rapidly removed at 4°C. The cortex tissue were homogenized in 30 volumes of ice-cold 50mM Tris-HCl buffer(pH7.4) using an ultra-turrax T8 and centrifuged at 48,000 g for 20 min. The supernatant was discarded and the pellet washed twice by resuspension in 20 volumes Tris-HCl and centrifugation, homogenized in 20 volumes Tris-HCl, and incubated at 37 °C for 10 min. Membranes were than collected by centrifugation and washed twice before final resuspension in 10 volumes of 50 mM Tris-HCl, pH7.4.
For binding assays, the incubation medium consisted of 162.5ug of tissue homogenate, 0.92nM of [3H]8-OH-DPAT and vehicle or 10uM of 5-HT or 10nM of test compounds per well. 5-HT and test compounds were dissolved in distilled water and incubation medium was 50mM Tris (pH7.4 at room temperature). The assay plates(96well) were incubated for 60 min at 25 °C. The reaction was terminated by rapid filtration using a Tomtec harvester, through GF/C glassfibre filter with 5 times washes of Tris buffer. The radioactivity retained on the filters was counted by scintillation spectroscopy (MicroBeta TriLux, Wallac). The tests were triplicates. Percent inhibition was calculated as below equation.

$$\%\ \text{inhibition} = \frac{\text{Total binding - Compound binding}}{\text{Specific(Total - Nonspecific) binding}}$$

Total binding is the value of vehicle treated group and specific binding is the values that deduct non-specific binding (i.e. the value of non-specific ligand, 5-HT).
The results obtained by testing compounds of the invention are given in the following Table 1.

**Table 1**

| Test Compound | 5-HT1a % inhibition, at 10nM |
|---|---|
| Example 1 | 83.1 |
| Example 2 | 80.3 |
| Example 3 | 77.8 |
| Example 4 | 75.9 |
| Example 5 | 50.7 |
| Example 6 | 50.1 |
| Example 7 | 78.4 |
| Example 8 | 81.6 |
| Example 9 | 52.2 |
| Example 10 | 71.8 |
| Example 11 | 61.8 |
| Example 12 | 60.9 |
| Example 13 | 50.4 |
| Example 14 | 51.1 |
| Example 15 | 23.5 |
| Example 16 | 62.5 |
| Example 17 | 56.4 |
| Example 18 | 69.5 |
| Example 19 | 59.6 |
| Example 20 | 68.5 |
| Example 21 | 38.2 |
| Example 22 | 53.4 |
| Example 23 | 17.2 |
| Example 24 | 51.6 |
| Example 25 | 45.2 |
| Example 26 | 47.0 |
| Example 27 | 40.9 |
| Example 28 | 13.5 |
| Example 29 | 45.8 |
| Example 30 | 38.3 |
| Example 31 | 30.3 |
| Example 33 | 53.7 |
| Example 35 | 73.3 |
| Example 36 | 28.1 |
| Example 37 | 24.8 |
| Example 38 | 29.4 |
| Example 39 | 52.9 |
| Example 40 | 45.6 |

(continued)

| Test Compound | 5-HT1a % inhibition, at 10nM |
|---|---|
| Example 41 | 82.1 |
| Example 42 | 36.2 |
| Example 43 | 95.6 |
| Example 44 | 17.4 |
| Example 45 | 73.8 |
| Example 46 | 64.6 |
| Example 47 | 70.0 |
| Example 48 | 48.0 |
| Example 49 | 45.0 |
| Example 50 | 51.0 |
| Example 51 | 47.0 |
| Example 52 | 62.0 |
| Example 53 | 46.0 |
| Example 54 | 71.0 |
| Example 56 | 24.3 |

**Example 58: Tail Suspension Test in mice (TST)**

Tail Suspension Test in mice is the well-known animal model to determine antidepressant efficacy of compounds by checking escaping behavior and immobility time of mice. Mice treated with antidepressant drug show escaping behavior continuously comparing with control group.

The mice were mainly treated orally with the test compound dissolved in 30% PEG400 or with only 30% PEG400 as control. Tail of mouse is suspended to the rod of TST equipment and end of tail is fixed with sticky tape. Suspended mouse is incubated during 2 min for training and adaptation and after fallowing 2min, observe escaping behavior and check immobility time for 4min. The potent ability of the compounds was determined as percent value of reduction in immobility comparing to control group as 100% baseline.

The results obtained by testing compounds of the invention are given in the following Table 2:

**Table 2**

| Test Compound | % reduction at 1mg/kg, po |
|---|---|
| Example 5 | 21.2 |
| Example 7 | 21.1 |
| Example 8 | 19.6 |
| Example 11 | 25 |
| Example 12 | 12.7 |
| Example 18 | 14.7 |
| Example 19 | 50.6 |
| Example 33 | 26.8 |
| Example 39 | 30.4 |
| Example 40 | 10.6 |
| Example 41 | 14.1 |
| Example 48 | 26.2 |

(continued)

| Test Compound | % reduction at 1mg/kg, po |
|---|---|
| Example 49 | 34.3 |
| Example 50 | 46.9 |
| Example 51 | 14.7 |
| Example 52 | 26.4 |
| Example 54 | 31.8 |
| Estalopram | 47, at 30mg/kg, po |
| Fluxetine | 49. at 30mg/kg, po |

This anti-depressant effect, as with other anti-depressant drugs, suggested potentials for treating depression.

**Example 59: Anti-Marble Burying Behavior Test in Mice**

Marble burying was developed and validated as a pre-clinical assay of potential anxiolytic activity (Andrews and Broekkamp (1993). Procedures to Identify Anxiolytic or Anxiogenic agents. In Behavioural Neuroscience, ed. A Sahgal, pp. 37-54. IRL Press, Oxford). The marble burying test places a naive mouse into a novel environment containing 25 marbles (arranged on top of a sawdust surface). A reduction in the number of marbles buried by the mouse was hypothesized to be an anxiolytic-like effect.

The results obtained by testing compounds of the invention are given in the following Table 3:

**Table 3**

| Test Compound | % reduction, at 3mg/kg,ip |
|---|---|
| Example 1 | 28.7 |
| Example 2 | 93.3 |
| Example 5 | 60.3, at 7.5mg/kg, ip |
| Example 7 | 61.1, at 7.5mg/kg, ip |
| Example 11 | 69.0, at 7.5mg/kg, ip |
| Example 12 | 97.3 |
| Example 19 | 50.8, at 7.5mg/kg, ip |
| Example 20 | 27.0, at 7.5mg/kg, ip |
| Example 21 | 29.9 |
| Example 24 | 62.6 |
| Example 28 | 24.6 |
| Example 32 | 27.8, at 7.5mg/kg, ip |
| Example 36 | 25.3 |
| Example 47 | 39.3 |
| Example 48 | 31.3 |
| Example 50 | 96.8, at 7.5mg/kg, ip |
| Example 51 | 44.4, at 7.5mg/kg, ip |
| Buspirone | 56.0, at 20mg/kg, ip |

An anti-marble burying effect, as with other anti-anxiety drugs, suggested potentials for treating anxiety.

## Claims

**1.** A piperazine compound represented by Formula (I):

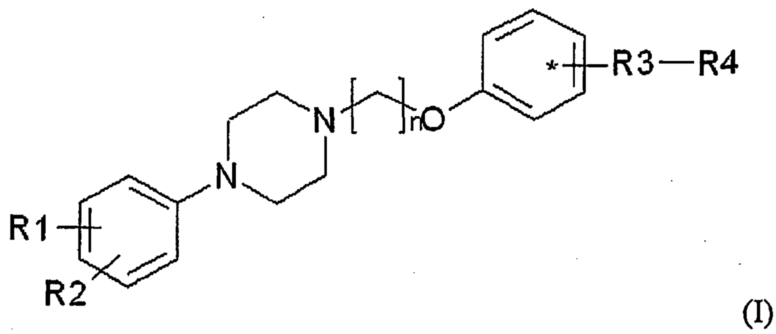

(I)

or pharmaceutically acceptable salts thereof, wherein

n is an integer of 2 to 6;
R1 and R2 are the same or different and are independently selected from the group consisting of hydrogen, a hydroxyl group, a halogen, nitrogen dioxide, a straight or branched chain alkyl group with 1 to 4 carbon atoms, and a straight or branched chain alkoxy group with 1 to 4 carbon atoms;
R3 is a C1-C2 alkylene; and
R4 is selected from the group consisting of:

(a) a C2-C6 dialkylamine,
(b) a 5 to 9-membered aromatic amine wherein the ring is independently substituted with at least one of hydrogen, a C1-C6 aliphatic alkyl, a C3 to C10 cyclic alkyl, a C6-C10 aryl group and a halogen,
(c) a 5 to 9-membered heteroaromatic amine comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen,
(d) a 5 to 9-membered heterocyclic ring comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen, and
(e) a 5 to 9-membered heteroaromatic ring comprising at least a nitrogen atom as a ring constituent wherein the ring is independently substituted with at least one of hydrogen, a C1-C6 aliphatic alkyl, a C3 to C10 cyclic alkyl, a C6-C10 aryl group and a halogen; and
R4 is connected to R3 group through a nitrogen atom therein.

**2.** The piperazine compound of Claim 1, wherein R4 is dimethylamine, or diethylamine.

**3.** The piperazine compound of Claim 1, wherein R4 is connected to R3 through a nitrogen atom in an amino group of the 5 to 9-membered aromatic amine or heteroaromatic amine.

**4.** The piperazine compound of Claim 3, wherein R4 is a 5 or 6-membered aromatic amine substituted with at least one of hydrogen, a C1-C6 alkyl, and a halogen.

**5.** The piperazine compound of Claim 4, wherein R4 is a 6-membered aromatic amine substituted with hydrogen.

**6.** The piperazine compound of Claim 3, wherein R4 is a 5 or 6-membered heteroaromatic amine substituted with at least one group selected from the group consisting of a C1-C6 alkyl and a halogen, wherein the heteroaromatic amine comprises at least two heteroatoms as ring constituents where a first heteroatom is N and a second heteroatom is independently selected from the group consisting of N, O, and S.

**7.** The piperazine compound of Claim 6, wherein R4 is a 5-membered heteroaromatic amine having a structure (II) selected from the group consisting of:

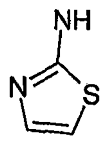 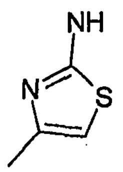 and 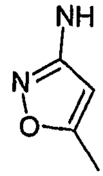 (II)

8. The piperazine compound of Claim 1, wherein R4 is connected to R3 through a nitrogen atom contained in a ring as a heteroatom of the 5 to 9-membered heterocyclic ring or heteroaromatic ring.

9. The piperazine compound of Claim 8, wherein R4 is a 5 or 6-membered heterocyclic ring having a structure (III) selected from the group consisting of:

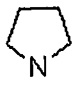 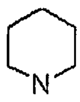 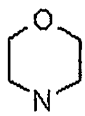 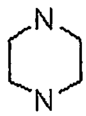 and 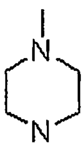 (III)

10. The piperazine compound of Claim 8, wherein R4 is an selected from the group consisting of diazole, triazole, tetraazol, benzotriazole, imidazole, pyrazole, benzimidazloe, and indazole.

11. The piperazine compound of Claim 10, wherein the azole has a structure (IV) selected from the group consisting:

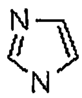 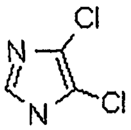 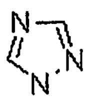 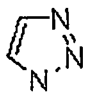 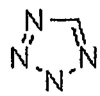 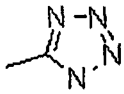

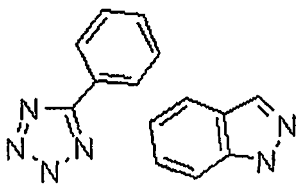 and 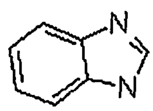 (IV)

12. The piperazine compound of Claim 1, wherein R1 and R2 are independently selected from the group consisting of hydrogen, a halogen, a straight or branched chain alkyl group with 1 to 4 carbon atoms, and a straight or branched chain alkoxy group with 1 to 4 carbon atoms.

13. The piperazine compound of Claim 1, wherein said compound is selected from the group consisting of: 1-[4-(4-imidazol-1-ylmethyl-phenoxy)-butyl]-4-(2-methoxy-phenyl)-piperazine, 1-(2-methoxy-phenyl)-4-[4-(4-[1,2,4]txiazol-1-ylmethyl-phenoxy)-butyl]-piperazine, 1-(2-methoxy-phenyl)-4-[4-(4-tetrazol-1-ylmethyl-phenoxy)-butyl]-piperazine, 1-(2-methoxy-phenyl)-4-[4-(4-tetrazol-2-ylmethyl-phenoxy)-butyl]-piperazine, 1-[4-(3-imidazol-1-ylmethyl-phenoxy)-butyl]-4-(2-methoxy-phenyl)-piperazine- hydrochloride, 1-(2-methoxy-phenyl)-4-[4-(3-[1,2,4]triazol-1-ylmethyl-phenoxy)-butyl]-piperazine hydrochloride, 1-(2-methoxy-phenyl)-4-[4-(3-tetrazol-1-ylmethyl-phenoxy)-butyl]-piperazine hydrochloride, 1-(2-methoxy-phenyl)-4-[4-(3-tetrazol-2-ylmethyl-phenoxy)-butyl]-piperazine hydrochloride, 1-(4-{4-[4-(2-methoxy-phenyl)-piperazin-1-yl]-butoxy}-benzyl)-1H-benzoimidazole, 1-(2-Methoxy-phenyl)-4-{4-[4-(2-methyl-imidazol-1-ylmethyl)-phenoxy]-butyl}-piperazine, 1-(2-methoxy-phenyl)-4-[4-(4-[1,2,3]triazol-2-ylmethyl-phenoxy)-butyl]-piperazine, 1-(2-methoxy-phenyl)-4-[4-(4-[1,2,3]triazol-1-ylmethyl-phenoxy)-butyl]-piperazine, 1-(4-{4-[4-(2-Methoxy-phenyl)-piperazin-1-yl]-butoxy}-benzyl)-1H-indazole, 1-(4-{4-[4-(2-Methoxy-phenyl)-piperazin-1-yl]-butoxy}-benzyl)-1H-benzotriazole, 1-(2-methoxy-phenyl)-4-{4-[4-(5-phenyl-tetrazol-2-ylmethyl)-phenoxy]-butyl}-piperazine, 1-(2-methoxy-phenyl)-4-{4-[4-(5-phenyl-tetrazol-1-ylmethyl)-phenoxy]-butyl}-piperazine, 1-(2-methoxy-phenyl)-4-{4-[4-(5-methyl-tetrazol-2-ylmethyl)-phenoxy]-butyl}-piperazine, 1-(2-methoxy-phenyl)-4-{4-[4-(5-methyl-tetrazol-1-ylmethyl)-phenoxy]-butyl}-piperazine,(4-{4-[4-(2-methoxy-phenyl)-piperazin-1-yl]-butoxy}-benzyl)-dimethyl-amine di-hydrochloride, 1-(2-methoxy-phenyl)-4-[4-(4-piperidin-1-ylmethyl-phenoxy)-butyl]-piperazine, (4-{4-[4-(2-methoxy-phenyl)-piperazin-1-yl]-butoxy}-benzyl)-phenyl-amine, (4-{4-[4-(2-methoxy-phenyl)-piperazin-1-yl]-butoxy}-benzyl)-thiazol-2-yl-amine, 1-(2-chloro-phenyl)-4-[4-(4-[1,2,4]triazol-1-ylmethyl-phenoxy)-butyl]-piperazine, 1-(2-methoxy-phenyl)-4-[4-(3-[1,2,3]triazol-2-ylmethyl-phenoxy)-butyl]-piperazine, 1-(2-Methoxy-phenyl)-4-[4-(3-[1,2,3]triazol-1-ylmethyl-phenoxy)-butyl]-piperazine, 1-(2-Methoxy-phenyl)-4-[4-(3-[1,2,3]triazol-2-ylmethyl-phenoxy)-butyl]-piperazine, 1-(2-methoxy-phenyl)-4-{4-[3-(5-methyl-tetrazol-1-ylmethyl)-phenoxy]-butyl}-piperazine, (3-{4-[4-(2-methoxy-phenyl)-piperazin-1-yl]-butoxy}-benzyl)-dimethyl-amine, 4-(3-{4-[4-(2-methoxy-phenyl)-piperazin-1-yl]-butoxy}-benzyl)-morpholine, 1-{4-[4-(4,5-dichloro-imidazol-1-ylmethyl)-phenoxy]-butyl}-4-(2-methoxy-phenyl)-piperazine, 1-(2-chloro-phenyl)-4-[4-(4-tetrazol-2-ylmethyl-phenoxy)-butyl]-piperazine, 4-(4-{4-[4-(2-methoxy-phenyl)-piperazin-1-yl]-butoxy}-benzyl)-morpholine, (4-{4-[4-(2-Chloro-phenyl)-piperazin-1-yl]-butoxy}-benzyl)-dimethyl-amine, 1-(3-fluoro-phenyl)-4-[4-(4-[1,2,4]triazol-1-ylmethyl-phenoxy)-butyl]-piperazine, 1-(2-methoxy-phenyl)-4-[4-(4-4-methylpiperazine-1-ylmethyl-phenoxy)-butyl]-piperazine, , 1-(2,3-dichloro-phenyl)-4-[4-(4-tetrazol-2-ylmethyl-phenoxy)-butyl]-piperazine, 1-(2,3-Dichloro-phenyl)-4-{4-[4-(5-methyl-tetrazol-1-ylmethyl)-phenoxy]-butyl}-piperazine, 1-(2,3-Dichloro-phenyl)-4-{4-[4-(5-methyl-tetrazol-2-ylmethyl)-phenoxy]-butyl}-piperazine, (4-{4-[4-(2,3-dichloro-phenyl)-piperazin-1-yl]-butoxy}-benzyl)-dimethyl-amine, 4-(4-{4-[4-(2,3-Dichloro-phenyl)-piperazin-1-yl]-butoxy}-benzyl)-morpholine, 1-(2-methoxy-phenyl)-4-[4-(4-4-methylpiperazine-1-ylmethyl-phenoxy)-butyl]-piperazine, 1-(2-chlorophenyl)-4-[4-(4-[1,2,3]triazol-1-ylmethyl-phenoxy)-butyl]-piperazine, (4-{4-[4-(2-Methoxy-phenyl)-piperazin-1-yl]-butoxy}-benzyl)-[1,3,4]thiadiazol-2-yl-amine, 1-(2,3-dichloro-phenyl)-4-[4-(4-[1,2,3]triazol-1-ylmethyl-phenoxy)-butyl]-piperazine, (4-{4-[4-(2-Methoxy-phenyl)-piperazin-1-yl]-butoxy} -benzyl)-(5-methyl-isoxazol-3-yl)-amine, (4-{4-[4-(2-methoxy-phenyl)-piperazin-1-yl]-butoxy}-benzyl)-(5-methyl-isoxazol-3-yl)-amine, 1-{4-[4-(2-imidazol-1-yl-ethyl)-phenoxy]-butyl}-4-(2-methoxy-phenyl)-piperazine, 1-(2-methoxy-phenyl)-4-{4-[4-(2-[1,2,4]triazol-1-yl-ethyl)-phenoxy]-butyl}-piperazine, 1-(2-methoxy-phenyl)-4-{4-[4-(2-[1,2,3]triazol-2-yl-ethyl)-phenoxy]-butyl}-piperazine, 1-(2-methoxy-phenyl)-4-{4-[4-(2-[1,2,3]triazol-1-yl-ethyl)-phenoxy]-butyl}-piperazine, 1-(2-methoxy-phenyl)-4-{4-[4-(2-tetrazol-2-yl-ethyl)-phenoxy]-butyl}-piperazine, 1-(2-methoxy-phenyl)-4-{4-[4-(2-tetrazol-1-yl-ethyl)-phenoxy]-butyl}-piperazine, 1-(2-Methoxy-phenyl)-4-(4-{4-[2-(5-methyl-tetrazol-1-yl)-ethyl]-phenoxy}-butyl)-piperazine, 1-(2-Methoxy-phenyl)-4-(4-{4-[2-(5-methyl-tetrazol-2-yl)-ethyl]-phenoxy}-butyl)-piperazine, 2-{4-[4-(4-[1,2,3]-triazol-1-ylmethyl-phenoxy)-butyl]-piperazin-1-yl}-benzonitrile, and 1-(2-chloro-phenyl)-4-[4-(4-[1,2,3]triazol-2-ylmethyl-phenoxy)-butyl]-piperazine.

14. A pharmaceutical composition for treating and preventing depression and anxiety comprising an effective amount of the piperazine compound of Claims 1 to 13, and a pharmaceutically acceptable carrier or excipient.

**Patentansprüche**

1. Piperazinverbindung, dargestellt durch die folgende Formel (I):

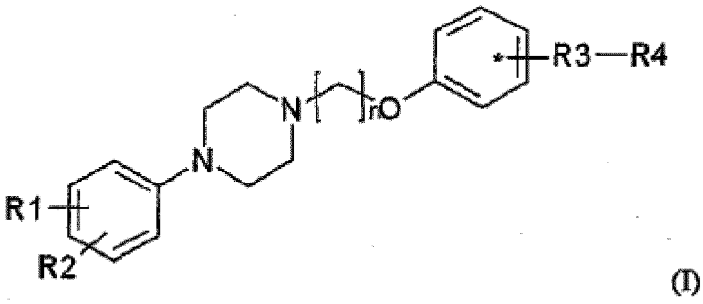

(I)

oder pharmazeutisch unbedenkliche Salze davon, wobei

n eine ganze Zahl von 2 bis 6 ist;
R1 und R2 gleich oder verschieden sind und unabhängig aus der Gruppe bestehend aus Wasserstoff, einer Hydroxylgruppe, einem Halogen, Stickstoffdioxid, einer gerad- oder verzweigtkettigen Alkylgruppe mit 1 bis 4 Kohlenstoffatomen und einer gerad- oder verzweigtkettigen Alkoxygruppe mit 1 bis 4 Kohlenstoffatomen ausgewählt sind;
R3 ein C1-C2-Alkylen ist; und

R4 ausgewählt ist aus der Gruppe bestehend aus:

(a) einem C2-C6-Dialkylamin,
(b) einem 5- bis 9-gliedrigen aromatischen Amin, wobei der Ring unabhängig mit mindestens einem von Wasserstoff, einem aliphatischen C1-C6-Alkyl, einem cyclischen C3- bis C10-Alkyl, einer C6-C10-Arylgruppe und einem Halogen substituiert ist,
(c) einem 5 - bis 9-gliedrigen heteroaromatischen Amin, das mindestens ein Stickstoffatom als einen Ringbestandteil umfasst, wobei der Ring unabhängig mit mindestens einem von Wasserstoff, einem aliphatischen C1-C6-Alkyl, einem cyclischen C3- bis C10-Alkyl, einer C6-C10-Alkylgruppe und einem Halogen substituiert ist,
(d) einem 5- bis 9-gliedrigen heterocyclischen Ring, der mindestens ein Stickstoffatom als einen Ringbestandteil umfasst, wobei der Ring unabhängig mit mindestens einem von Wasserstoff, einem aliphatischen C1-C6-Alkyl, einem cyclischen C3- bis C10-Alkyl, einer C6-C10-Arylgruppe und einem Halogen substituiert ist, und
(e) einem 5- bis 9-gliedrigen heteroaromatischen Ring, der mindestens ein Stickstoffatom als einen Ringbestandteil umfasst, wobei der Ring unabhängig mit mindestens einem von Wasserstoff, einem aliphatischen C1-C6-Alkyl, einem cyclischen C3- bis C10-Alkyl, einer C6-C10-Arylgruppe und einem Halogen substituiert ist, und
R4 mit der R3-Gruppe durch ein Stickstoffatom darin verbunden ist.

**2.** Piperazinverbindung nach Anspruch 1, wobei R4 Dimethylamin oder Diethylamin ist.

**3.** Piperazinverbindung nach Anspruch 1, wobei R4 mit R3 durch ein Stickstoffatom in einer Aminogruppe des 5- bis 9-gliedrigen aromatischen Amins oder heteroaromatischen Amins verbunden ist.

**4.** Piperazinverbindung nach Anspruch 3, wobei R4 ein 5- oder 6-gliedriges aromatisches Amin ist, das mit mindestens einem von Wasserstoff, einem C1-C6-Alkyl und einem Halogen substituiert ist.

**5.** Piperazinverbindung nach Anspruch 4, wobei R4 ein 6-gliedriges aromatisches Amin ist, das mit Wasserstoff substituiert ist.

**6.** Piperazinverbindung nach Anspruch 3, wobei R4 ein 5- oder 6-gliedriges heteroaromatisches Amin ist, das mit mindestens einer Gruppe substituiert ist, die aus der Gruppe bestehend aus einem C1-C6-Alkyl und einem Halogen ausgewählt ist, wobei das heteroaromatische Amin mindestens zwei Heteroatome als Ringbestandteile umfasst, wobei ein erstes Heteroatom N ist, und ein zweites Heteroatom unabhängig aus der Gruppe bestehend aus N, O und S ausgewählt ist.

**7.** Piperazinverbindung nach Anspruch 6, wobei R4 ein 5-gliedriges heteroaromatisches Amin mit einer Struktur (II) ist, die ausgewählt ist aus der Gruppe bestehend aus:

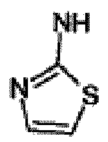
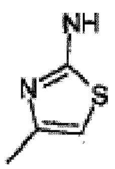
und
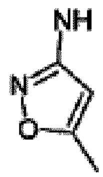
(II) .

**8.** Piperazinverbindung nach Anspruch 1, wobei R4 durch ein Stickstoffatom, das in einem Ring als ein Heteroatom des 5- bis 9-gliedrigen heterocyclischen Rings oder heteroaromatischen Rings enthalten ist, mit R3 verbunden ist.

**9.** Piperazinverbindung nach Anspruch 8, wobei R4 ein 5- oder 6-gliedriger heterocyclischer Ring mit einer Struktur (III) ist, die ausgewählt ist aus der Gruppe bestehend aus:

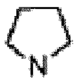 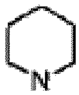 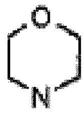 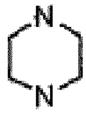 und 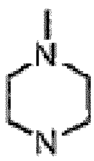 (III).

**10.** Piperazinverbindung nach Anspruch 8, wobei R4 ein Azol ist, das aus der Gruppe bestehend aus Diazol, Triazol, Tetrazol, Benzotriazol, Imidazol, Pyrazol, Benzimidazol und Indazol ausgewählt ist.

**11.** Piperazinverbindung nach Anspruch 10, wobei das Azol eine Struktur (1V) aufweist, die ausgewählt ist aus der Gruppe bestehend aus:

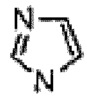 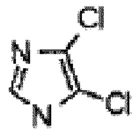 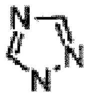 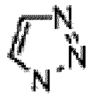 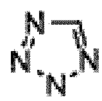 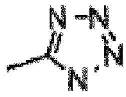

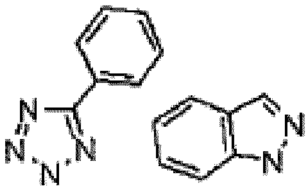 und 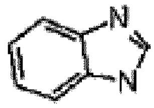 (IV).

**12.** Piperazinverbindung nach Anspruch 1, wobei R1 und R2 unabhängig aus der Gruppe bestehend aus Wasserstoff, einer Hydroxylgruppe, einem Halogen, einer gerad- oder verzweigtkettigen Alkylgruppe mit 1 bis 4 Kohlenstoffatomen und einer gerad- oder verzweigtkettigen Alkoxygruppe mit 1 bis 4 Kohlenstoffatomen ausgewählt sind.

**13.** Piperazinverbindung nach Anspruch 1, wobei die Verbindung ausgewählt ist aus der Gruppe bestehend aus:

1-[4-(4-Imidazol-1-ylmethyl-phenoxy)-butyl]-4-(2-methoxy-phenyl)-piperazin,
1-((2-Methoxy-phenyl)-4[4-(4-[1,2,4]triazol-1-ylmethyl-phenoxy)-butyl]-piperazin,
1-((2-Methoxy-phenyl)-4[4-(4-tetrazol-1-ylmethyl-phenoxy)-butyl]-piperazin,
1-((2-Methoxy-phenyl)-4[4-(4-tetrazol-2-ylmethyl-phenoxy)-butyl]-piperazin,
1-[4-(3-Imidazol-1-ylmethyl-phenoxy)-butyl]-4-(2-methoxy-phenyl)-piperazin-hydrochlorid,
1-((2-Methoxy-phenyl)-4[4-(3-[1,2,4]triazol-1-ylmethyl-phenoxy)-butyl]-piperazin-hydrochlorid,
1-((2-Methoxy-phenyl)-4-[4-(3-tetrazol-1-ylmethyl-phenoxy)-butyl]-piperazin-hydrochlorid,
1-((2-methoxy-phenyl)-4[4-(3-tetrazol-2-ylmethyl-phenoxy)-butyl]-piperazin-hydrochlorid,
1-((4-{4-{4-(2-Methoxy-phenyl)-piperazin-1-yl]-butoxy}-benzyl)-1H-benzoimidazol,
1-((2-Methoxy-phenyl)-4-{4-[4-(2-methyl-imidazol-1-ylmethyl)-phenoxy]-butyl}-piperazin,
1-((2-Methoxy-phenyl)-4-[4-(4-[1,2,3]triazo1-2-ylmethyl-phenoxy)-butyl]-piperazin,
1-((2-Methoxy-phenyl)-4[4-(4-[1,2,3]triazol-1-ylmethyl-phenoxy)-butyl]-piperazin,
1-((4-{4-[4-(2-Methoxy-phenyl)-piperazin-1-yl]-butoxy}-benzyl)-1H-indazol,
1-((4-{4-[4-(2-Methoxy-phenyl)-piperazin-1-yl]-butoxy}-benzyl)-1H-benzotriazol,
1-((2-Methoxy-phenyl)-4-{4-[4-(5-phenyl-tetrazol-2-ylmethyl)-phenoxy]-butyl}-piperazin,
1-((2-Methoxy-phenyl)-4-{4-[4-(5-phenyl-tetrazo1-1-ymethyl)-phenoxy]-butyl}-piperazin,
1-((2-Methoxy-phenyl)-4-{4-[4-(5-methyl-tetrazol-2-ylmethyl)-phenoxy]butyl}-piperazin,
1-((2-Methoxy-phenyl)-4-{4-[4-(5-methyl-tetrazol-1-ylmethyl)-phenoxy]-butyl}-piperazin,
(4-{4-[4-(2-Methoxy-phenyl)-piperazin-1-yl]-butoxy}-benzyl)-dimethyl-amin-di-hydrochlorid,
1-((2-Methoxy-phenyl)-4[4-(4-piperidin-1-ylmethyl-phenoxy)-butyl]-piperazin,
(4-{4-[4-(2-Methoxy-phenyl)-piperazin-1-yl]-butoxy}-benzyl)-phenyl-amin,
(4-{4-[4-(2-Methoxy-phenyl)-piperazin-1-yl]-butoxy}-benzyl)-thiazol-2-yl-amin,
1-((2-Chlor-phenyl)-4[4-(4-[1,2,4]triazol-1-ylmethyl-phenoxy)-butyl]-piperazin,

1-((2-Methoxy-phenyl)-4[4-(3-[1,2,3]triazol-2-ylmethyl-phenoxy)-butyl]-piperazin,
1-((2-Methoxy-phenyl)-4[4-(3-[1,2,3]triazol-1-ylmethyl-phenoxy)-butyl]-piperazin,
1-((2-Methoxy-phenyl)-4[4-(3-[1,2,3]triazol-2-ylmethyl-phenoxy)-butyl]-piperazin,
1-((2-Methoxy-phenyl)-4-{4-[3-(5-methyl-tetrazol-1-ylmethyl)-phenoxylbutyl-piperazin,
(3-{4-[4-(2-Methoxy-phenyl)-piperazin-1-yl]-butoxy}-benzyl)-dimethyl-amin,
4-((3-{4-{4-(2-Methoxy-phenyl)-piperazin-1-yl]-butoxy}-benzyl)-morpholin,
1-{4-[4-(4,5-Dichlor-imidazol-1-ylmethyl)-phenoxyl-butyl}-4-(2-methoxy-phenyl)-piperazin,
1-((2-Chlor-phenyl)-4-[4-(4-tetrazol-2-ylmethyl-phenoxy)-butyl]-piperazin,
4-((4-{4-[4-(2-Methoxy-phenyl)-piperazin-1-yl]-butoxy}-benzyl)-morpholin,
(4-{4-[4-(2-Chlor-phenyl)-piperazin-1-yl]-butoxy}-benzyl)-dimethyl-amin,
1-((3-Fluor-phenyl)-4[4-(4-[1,2,4]triazol-1-ylmethyl-phenoxy)-butyl]piperazin,
1-((2-Methoxy-phenyl)-4[4-(4-4-methylpiperazin-1-ylmethyl-phenoxy)-butyl]-piperazin,
1-((2,3-Dichlor-phenyl)-4-[4-(4-tetrazol-2-ylmethyl-phenoxy)-butyl]piperazin,
1-((2,3-Dichlor-phenyl)-4-{4-[4-(5-methyl-tetrazol-1-ylmethyl)-phenoxy]-butyl}-piperazin,
1-((2,3-Dichlor-phenyl)-4-{4[4-(5-methyl-tetrazol-2-ylmethyl)-phenoxy]-butyl}-piperazin,
(4-{4-[4-(2,3-Dichlor-phenyl)-piperazin-1-yl]-butoxy}-benzyl)-dimethyl-amin,
4-((4-{4-[4-(2,3-Dichlor-phenyl)-piperazin-1-yl]-butoxy}-benzyl)-morpholin, 1-(2-Methoxy-phenyl)-4[4-(4-4-methylpiperazin-1-ylmethyl-phenoxy)-butyl]-piperazin,
1-((2-Chlor-phenyl)-4[4-(4-[1,2,3]triazol-1-ylmethyl-phenoxy)-butyl]piperazin,
(4-{4-[4-[2-Methoxy-phenyl)-piperazin-1-yl]-butoxy}-benzyl)-[1,3,4]thiadiazol-2-yl-amin,
1-((2,3-Dichlor-phenyl)-4[4-(4-[1,2,3)triazol-1-ylmethyl-phenoxy)-butyl]-piperazin,
(4-{4-[4-(2-Methoxy-phenyl)-piperazin-1-yl]-butoxy}-benzyl)-(5-methyl-isoxazol-3-yl)-amin,
(4-{4-[4-(2-Methoxy-phenyl)-piperazin-1-yl]-butoxy}-benzyl)-(5-methyl-isoxazol-3-yl)-amin,
1-{4-[4-(2-Imidazol-1-yl-ethyl)-phenoxy]-butyl}-4-(2-methoxy-phenyl)-piperazin,
1-((2-Methoxy-phenyl)-4-{4-[4-(2-[1,2,4]triazol-1-yl-ethyl)-phenoxy]-butyl}-piperazin,
1-((2-Methoxy-phenyl)-4-{4-[4-(2-[1,2,3]triazol-2-yl-ethyl)-phenoxy]butyl}-piperazin,
1-((2-Methoxy-phenyl)-4-{4-[4-(2-[1,2,3]triazol-1-yl-ethyl)-phenoxyl-butyl}-piperazin,
1-((2-Methoxy-phenyl)-4-{4-[4-(2-tetrazol-2-yl-ethyl)-phenoxy]-butyl}-piperazin,
1-((2-Methoxy-phenyl)-4-{4-[4-(2-tetrazol-1-yl-ethyl)-phenoxy]-butyl}-piperazin,
1-((2-Methoxy-phenyl)-4-(4-{4-[2-(5-methyl-tetrazol-1-yl)-ethyl]-phenoxy}-butyl)-piperazin,
1-((2-Methoxy-phenyl)-4-(4-{4-[2-(5-methyl-tetrazol-2-yl)-ethyl]-phenoxy}-butyl)-piperazin,
2-{4-[4-(4-[1,2,3]-Triazol-1-ylmethyl-phenoxy)-butyl]-piperazin-1-yl}-benzonitril, und
1-((2-Chlor-phenyl)-4[4-(4-[1,2,3]triazol-2-ylmethyl-phenoxy)-butyl]-piperazin.

14. Pharmazeutische Zusammensetzung zur Behandlung und Verhütung von Depression und Angstgefühl, umfassend eine wirksame Menge der Piperazinverbindung nach Anspruch 1 bis 13 und einen pharmazeutisch unbedenklichen Träger oder Exzipienten.

## Revendications

1. Composé pipérazine représenté par la formule (I) :

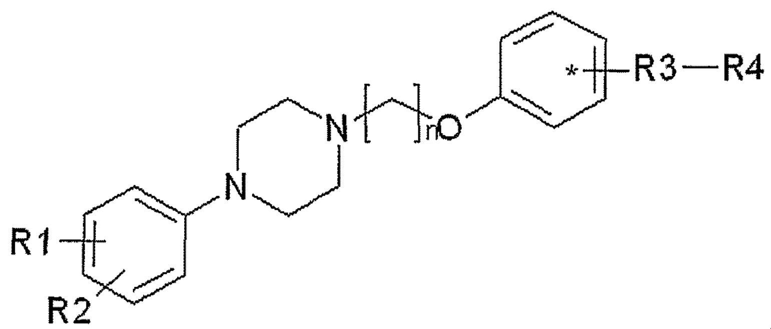

(I)

ou ses sels pharmaceutiquement acceptables, formule dans laquelle

n est un entier de 2 à 6 ;
R1 et R2 sont identiques ou différents et sont indépendamment choisis dans l'ensemble constitué par l'hydrogène, un groupe hydroxyle, un halogène, le dioxyde d'azote, un groupe alkyle à chaîne droite ou ramifiée ayant 1 à 4 atomes de carbone, et un groupe alcoxy à chaîne droite ou ramifiée ayant 1 à 4 atomes de carbone ;
R3 est un alkylène en C1-C2 ; et
R4 est choisi dans le groupe constitué par :

(a) une dialkylamine en C2-C6,
(b) une amine aromatique à 5 à 9 chaînons dans laquelle le cycle est indépendamment substitué par au moins l'un parmi l'hydrogène, un alkyle aliphatique en C1-C6, un alkyle cyclique en C3-C10, un groupe aryle en C6-C10 et un halogène,
(c) une amine hétéroaromatique à 5 à 9 chaînons comprenant au moins un atome d'azote à titre de constituant du cycle, dans laquelle le cycle est indépendamment substitué par au moins l'un parmi l'hydrogène, un alkyle aliphatique en C1-C6, un alkyle cyclique en C3-C10, un groupe aryle en C6-C10 et un halogène,
(d) un hétérocycle à 5 à 9 chaînons comprenant au moins un atome d'azote à titre de constituant du cycle, lequel cycle est indépendamment substitué par au moins l'un parmi l'hydrogène, un alkyle aliphatique en C1-C6, un alkyle cyclique en C3-C10, un groupe aryle en C6-C10 et un halogène, et
(e) un cycle hétéroaromatique à 5 à 9 chaînons comprenant au moins un atome d'azote à titre de constituant du cycle, lequel cycle est indépendamment substitué par au moins l'un parmi l'hydrogène, un alkyle aliphatique en C1-C6, un alkyle cyclique en C3-C10, un groupe aryle en C6-C10 et un halogène ; et
R4 est connecté au groupe R3 par l'intermédiaire d'un atome d'azote dans celui-ci.

**2.** Composé pipérazine selon la revendication 1, dans lequel R4 est la diméthylamine ou la diéthylamine.

**3.** Composé pipérazine selon la revendication 1, dans lequel R4 est connecté à R3 par l'intermédiaire d'un atome d'azote dans un groupe amino de l'amine aromatique ou de l'amine hétéroaromatique à 5 à 9 chaînons.

**4.** Composé pipérazine selon la revendication 3, dans lequel R4 est une amine aromatique à 5 ou 6 chaînons substituée par au moins l'un parmi l'hydrogène, un alkyle en C1-C6, et un halogène.

**5.** Composé pipérazine selon la revendication 4, dans laquelle R4 est une amine aromatique à 6 chaînons substituée par l'hydrogène.

**6.** Composé pipérazine selon la revendication 3, dans lequel R4 est une amine hétéroaromatique à 5 ou 6 chaînons substituée par au moins un groupe choisi dans l'ensemble constitué par un alkyle en C1-C6 et un halogène, et dans lequel l'amine hétéroaromatique comprend au moins deux hétéroatomes à titre de constituants du cycle, où un premier hétéroatome est N et un deuxième hétéroatome est indépendamment choisi dans l'ensemble constitué par N, O et S.

**7.** Composé pipérazine selon la revendication 6, dans lequel R4 est une amine hétéroaromatique à 5 chaînons de structure (II) choisie dans l'ensemble constitué par :

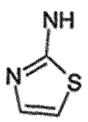 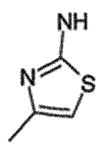 et 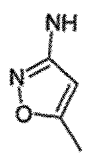 (II)

**8.** Composé pipérazine selon la revendication 1, dans lequel R4 est connecté à R3 par l'intermédiaire d'un atome d'azote contenu dans un cycle à titre d'hétéroatome du cycle hétéroaromatique ou de l'hétérocycle à 5 à 9 chaînons.

**9.** Composé pipérazine selon la revendication 8, dans lequel R4 est un hétérocycle à 5 ou 6 chaînons de structure (III) choisie dans l'ensemble constitué par :

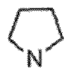 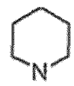 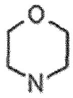 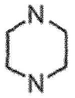 et 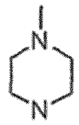 (III)

**10.** Composé pipérazine selon la revendication 8, dans lequel R4 est choisi dans l'ensemble constitué par le diazole, le triazole, le tétrazole, le benzotriazole, l'imidazole, le pyrazole, le benzimidazole, et l'indazole.

**11.** Composé pipérazine selon la revendication 10, dans lequel l'azole est de structure (IV) choisie dans l'ensemble constitué par :

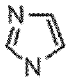 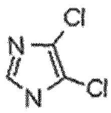 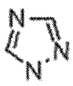 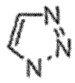 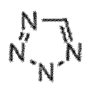 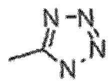

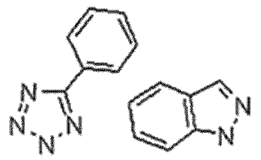 et 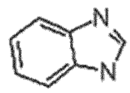

(IV)

**12.** Composé pipérazine selon la revendication 1, dans lequel R1 et R2 sont indépendamment choisis dans l'ensemble constitué par l'hydrogène, un halogène, un groupe alkyle à chaîne droite ou ramifiée ayant 1 à 4 atomes de carbone, et un groupe alcoxy à chaîne droite ou ramifiée ayant 1 à 4 atomes de carbone.

**13.** Composé pipérazine selon la revendication 1, lequel composé est choisi dans l'ensemble constitué par les suivants : 1-[4-(4-imidazol-1-ylméthylphénoxy)butyl]-4-(2-méthoxyphényl)pipérazine, 1-(2-méthoxyphényl)-4-[4-(4-[1,2,4]triazol-1-ylméthylphénoxy)butyl]pipérazine, 1-(2-méthoxyphényl)-4-[4-(4-tétrazol-1-ylméthylphénoxy)butyl]pipérazine, 1-(2-méthoxyphényl)-4-[4-(4-tétrazol-2-ylméthylphénoxy)butyl]pipérazine, chlorhydrate de 1-[4-(3-imidazol-1-ylméthylphénoxy)butyl]-4-(2-méthoxyphényl)pipérazine, chlorhydrate de 1-(2-méthoxyphényl)-4-[4-(3-[1,2,4]triazol-1-ylméthylphénoxy)butyl]pipérazine, chlorhydrate de 1-(2-méthoxyphényl)-4-[4-(3-tétrazol-1-ylméthylphénoxy)butyl]pipérazine, chlorhydrate de 1-(2-méthoxyphényl)-4-[4-(3-tétrazol-2-ylméthylphénoxy)butyl]pipérazine, 1-(4-{4-[4-(2-méthoxyphényl)pipérazin-1-yl]butoxy}benzyl)-1H-benzimidazole, 1-(2-méthoxyphényl)-4-{4-[4-(2-méthylimidazol-1-ylméthyl)phénoxy]butyl}pipérazine, 1-(2-méthoxyphényl)-4-[4-(4-[1,2,3]triazol-2-ylméthylphénoxy)butyl]pipérazine, 1-(2-méthoxyphényl)-4-[4-(4-[1,2,3]triazol-1-ylméthylphénoxy)butyl]pipérazine, 1-(4-{4-[4-(2-méthoxyphényl)pipérazin-1-yl]butoxy}benzyl)-1H-indazole, 1-(4-{4-[4-(2-méthoxyphényl)pipérazin-1-yl]butoxy}benzyl)-1H-benzotriazole, 1-(2-méthoxyphényl)-4-{4-[4-(5-phényltétrazol-2-ylméthyl)phénoxy]butyl}pipérazine, 1-(2-méthoxyphényl)-4-{4-[4-(5-phényltétrazol-1-ylméthyl)phénoxy]butyl}pipérazine, 1-(2-méthoxyphényl)-4-{4-[4-(5-méthyltétrazol-2-ylméthyl)phénoxy]butyl}pipérazine, 1-(2-méthoxyphényl)-4-{4-[4-(5-méthyltétrazol-1-ylméthyl)phénoxy]butyl}pipérazine, dichlorhydrate de (4-{4-[4-(2-méthoxyphényl)pipérazin-1-yl]butoxy}benzyl)diméthylamine, 1-(2-méthoxyphényl)-4-[4-(4-pipéridin-1-ylméthylphénoxy)butyl]pipérazine, (4-{4-[4-(2-méthoxyphényl)pipérazin-1-yl]butoxy}benzyl)phénylamine, (4-{4-[4-(2-méthoxyphényl)pipérazin-1-yl]butoxy}benzyl)thiazol-2-ylamine, 1-(2-chlorophényl)-4-[4-(4-[1,2,4]triazol-1-ylméthylphénoxy)butyl]pipérazine, 1-(2-méthoxyphényl)-4-[4-(3-[1,2,3]triazol-2-ylméthylphénoxy)butyl]pipérazine, 1-(2-méthoxyphényl)-4-[4-(3-[1,2,3]triazol-1-ylméthylphénoxy)butyl]pipérazine, 1-(2-méthoxyphényl)-4-[4-(3-[1,2,3]triazol-2-ylméthylphénoxy)butyl]pipérazine, 1-(2-méthoxyphényl)-4-{4-[3-(5-méthyltétrazol-1-ylméthyl)phénoxy]butyl}pipérazine, (3-{4-[4-(2-méthoxyphényl)pipérazin-1-yl]butoxy}benzyl)diméthylamine, 4-(3-{4-[4-(2-méthoxyphényl)pipérazin-1-yl]butoxy}benzyl)morpholine, 1-{4-[4-(4,5-dichloroimidazol-1-ylméthyl)phénoxy]butyl}-4-(2-méthoxyphényl)pipérazine, 1-(2-chlorophényl)-4-[4-(4-tétrazol-2-ylméthylphénoxy)butyl]pipérazine, 4-(4-{4-[4-(2-méthoxyphényl)pipérazin-1-yl]butoxy}benzyl)morpholine, (4-{4-[4-(2-chlorophényl)pipérazin-1-yl]butoxy}benzyl)diméthylamine, 1-(3-fluorophényl)-4-[4-(4-[1,2,4]triazol-1-ylméthylphénoxy)butyl]pipérazine, 1-(2-méthoxyphényl)-4-[4-(4-4-méthylpipérazine-1-ylméthylphénoxy)butyl]pipérazine, 1-(2,3-dichlorophényl)-4-[4-(4-tétrazol-2-ylméthylphénoxy)butyl]pipérazine, 1-(2,3-dichlorophényl)-4-{4-[4-(5-méthyltétrazol-1-ylméthyl)phénoxy]butyl}pipérazine, 1-(2,3-dichlorophényl)-4-{4-[4-(5-méthyltétrazol-2-ylméthyl)phénoxy]butyl}pipérazine, (4-{4-[4-(2,3-dichlorophényl)pipérazin-1-yl]butoxy}benzyl)diméthylamine, 4-(4-{4-[4-(2,3-dichlorophényl)pipérazin-1-yl]butoxy}benzyl)morpholine, 1-(2-méthoxyphényl)-4-[4-(4-4-méthylpipérazine-1-ylméthylphénoxy)butyl]pipérazine, 1-(2-chlorophényl)-4-[4-(4-[1,2,3]triazol-1-ylméthylphénoxy)butyl]pipérazine, (4-{4-[4-(2-méthoxyphényl)pipérazin-1-yl]butoxy}benzyl)-[1,3,4]thiadiazol-2-ylamine, 1-(2,3-dichlorophényl)-4-[4-(4-[1,2,3]triazol-1-ylméthylphénoxy)butyl]pipérazine, (4-{4-[4-(2-méthoxyphényl)pipérazin-1-yl]butoxy}benzyl)-(5-méthylisoxazol-3-yl)amine, (4-{4-[4-(2-méthoxyphényl)pipérazin-1-yl]butoxy}benzyl)-(5-méthylisoxazol-3-yl)amine, 1-{4-[4-(2-imidazol-1-yléthyl)phénoxy]butyl}-4-(2-méthoxyphényl)pipérazine, 1-(2-méthoxyphényl)-4-{4-[4-(2-[1,2,4]triazol-1-yléthyl)-phénoxy]butyl}pipérazine, 1-(2-méthoxyphényl)-4-{4-[4-(2-[1,2,3]triazol-2-yléthyl)phénoxy]butyl}pipérazine, 1-(2-méthoxyphényl)-4-{4-[4-(2-[1,2,3]triazol-1-yléthyl)phénoxy]butyl}pipérazine, 1-(2-méthoxyphényl)-4-{4-[4-(2-tétrazol-2-yléthyl)phénoxy]butyl}pipérazine, 1-(2-méthoxyphényl)-4-{4-[4-(2-tétrazol-1-yléthyl)phénoxy]butyl}pipérazine, 1-(2-méthoxyphényl)-4-(4-{4-[2-(5-méthyltétrazol-1-yl)éthyl]phénoxy}butyl)pipérazine, 1-(2-méthoxyphényl)-4-(4-{4-[2-(5-méthyltétrazol-2-yl)éthyl]phénoxy}butyl)pipérazine, 2-{4-[4-(4-[1,2,3]-triazol-1-ylméthylphénoxy)butyl]pipérazin-1-yl}benzonitrile, et 1-(2-chlorophényl)-4-[4-(4-[1,2,3]triazol-2-ylméthylphénoxy)butyl]pipérazine.

**14.** Composition pharmaceutique pour traiter et prévenir la dépression et l'anxiété, comprenant une quantité efficace du composé pipérazine des revendications 1 à 13, et un véhicule ou excipient pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5578596 A
- WO 9842692 A


### Non-patent literature cited in the description


- **KESSLER RC. et al.** *JAMA,* 2003, vol. 289, 3095-3105
- **HIRSCHFIELD RM. et al.** *JAMA,* 1997, vol. 277, 333-340
- **KELLER MB.** *J. Psychopharmacol.,* 1996, vol. 10 (1), 41-44
- **BLIER P. ; WARD M.** *Biol. Psychiatry,* 2003, vol. 53, 193-203
- **STAHL SM.** Essential Psychopharmacology. 2008, 531
- **ROBINSON DS. et al.** *J. Clin. Psychopharmacol.,* 1990, vol. 10 (3), 67S-76S
- **BIELSKI RJ.** *J. Clin. Psychiatry,* 2008, vol. 69 (4), 571-577
- **GROF P.** *International Clin. Psychopharmacology,* 1993, vol. 8 (3), 167-172
- Pharmaceutical Salts. *J. Pharm. Sci.,* 1977, vol. 66 (1), 1-19
- **HALL et al.** *J NeuroChem.,* 1985, vol. 44, 1685-1696
- Procedures to Identify Anxiolytic or Anxiogenic agents. **ANDREWS ; BROEKKAMP.** Behavioural Neuroscience. IRL Press, 1993, 37-54